# EUROPÄISCHE PATENTSCHRIFT

(11) **EP 3 642 252 B1**
(45) Veröffentlichungstag und Bekanntmachung des Hinweises auf die Patenterteilung: **08.12.2021**
(21) Anmeldenummer: 18730802.8
(22) Anmeldetag: 19.06.2018
(51) Int. Cl.: C08G 18/48, C08G 18/75, C08G 18/76, C09D 175/04, C08G 18/32, C09J 175/04, C08G 18/10, C08G 18/12

(54) **LATENTER HÄRTER UND HÄRTBARE POLYURETHANZUSAMMENSETZUNG**
LATENT CURING AGENT AND CURABLE POLYURETHANE COMPOSITION
DURCISSEUR LATENT ET COMPOSITION DE POLYURÉTHANE DURCISSABLE

(30) Priorität: 19.06.2017 EP 17176688
(43) Veröffentlichungstag der Anmeldung: 29.04.2020
(73) Patentinhaber: Sika Technology AG, 6340 Baar (CH)
(72) Erfinder: BURCKHARDT, Urs, 8049 Zürich (CH); KRAMER, Andreas, 8008 Zürich (CH)
(74) Vertreter: Sika Patent Attorneys
(86) Internationale Anmeldenummer: PCT/EP2018/066177
(87) Internationale Veröffentlichungsnummer: WO 2018/234267

(56) Entgegenhaltungen:
- WO-A2-2010/068736
- US-A1- 2015 111 991

## Beschreibung

### Technisches Gebiet

Die Erfindung betrifft latente Härter und härtbare Polyurethanzusammensetzungen enthaltend diese, sowie Klebstoffe, Dichtstoffe und Beschichtungen.

### Stand der Technik

Härtbare Polyurethanzusammensetzungen, welche durch Reaktion von Isocyanatgruppen mit Hydroxylgruppen und/oder Feuchtigkeit bzw. Wasser vernetzen, werden in vielen technischen Anwendungen eingesetzt, beispielsweise als Klebstoffe, Dichtstoffe oder Beschichtungen in der Bau- und Fertigungsindustrie. Wenn solche Zusammensetzungen bei hoher Luftfeuchtigkeit und/oder erhöhter Temperatur eingesetzt werden, entstehen bei der Aushärtung oft störende Blasen durch freigesetztes Kohlendioxid-Gas, das nicht schnell genug gelöst oder abgeleitet wird. Um die Blasenbildung zu vermeiden, können den Zusammensetzungen chemisch blockierte Amine, sogenannte latente Härter, zugegeben werden, welche bei Kontakt mit Feuchtigkeit Aminogruppen freisetzen und dabei rasch und ohne Kohlendioxid-Bildung mit den Isocyanatgruppen vernetzen. Als latente Härter werden meist Verbindungen mit Aldimin-, Ketimin- oder Oxazolidin-Gruppen eingesetzt. Die bekannten latenten Härter bringen aber Nachteile mit sich. So können sie vorzeitige Vernetzungsreaktionen auslösen und damit die Lagerstabilität der Zusammensetzungen herabsetzen und/oder deren Aushärtung so stark beschleunigen, dass sich eine zu kurze Offenzeit und damit ein zu kurzes Verarbeitungsfenster ergibt. Zudem führen viele der bekannten latenten Härter bei der Aushärtung zu störenden Immissionen, verursacht durch leichtflüchtige, geruchsintensive Aldehyde oder Ketone, welche im latenten Härter als Blockierungsmittel dienen und durch Hydrolyse freigesetzt werden.

Latente Härter mit Oxazolidin-Gruppen unterscheiden sich gegenüber solchen mit Aldimin- oder Ketimin-Gruppen dadurch, dass bei ihrer Hydrolyse nicht nur eine Aminogruppe, sondern zusätzlich eine Hydroxylgruppe entsteht, welche ebenfalls Isocyanat-reaktiv ist. Dies macht sie besonders ökonomisch im Verbrauch, führt bei der Aushärtung zu geringeren Mengen an freigesetztem Blockierungsmittel und erhöht die Vernetzungsdichte, was vor allem für Beschichtungen vorteilhaft ist. Allerdings haben sie auch Nachteile, die ihre Anwendung stark einschränken. Der kommerziell bekannteste Oxazolidin-Härter Incozol 4 ist schon bei oder wenig unterhalb von Raumtemperatur fest und muss deshalb aufgeschmolzen oder in einem Lösemittel gelöst werden. Zudem ist er mit vielen Isocyanaten nur sehr eingeschränkt lagerfähig und setzt bei der Aushärtung das hochflüchtige und geruchsintensive Isobutyraldehyd frei. US 5,189,176 beschreibt entsprechende Oxazolidin-Härter auf der Basis von 2-Ethylhexanal. Diese sind zwar bei Raumtemperatur flüssig, sind aber mit Isocyanaten ebenfalls nur eingeschränkt lagerfähig und führen zu sehr starken Geruchsimmissionen. Zudem ist 2-Ethylhexanal toxisch.

Weiterhin bekannt sind Oxazolidin-Härter abgeleitet von aromatischen Aldehyden, beispielsweise aus JP H08-034776 oder JP 2002-194048. Diese sind bei Raumtemperatur fest und führen zu starken Geruchsimmissionen und teilweise zu Verfärbungen.

Aus WO 2004/013088 sind geruchsfreie Aldimin-Härter bekannt, welche mit geruchsfreien tertiären aliphatischen, langkettigen und Estergruppen-haltigen Aldehyden blockiert sind. Oxazolidin-Härter auf Basis dieser Aldehyde wurden in WO 2010/068736 für die Anwendung in Heissschmelzklebstoffen vorgeschlagen. Sie sind mit allen Isocyanaten lagerstabil und verursachen keine Geruchsimmissionen, hydrolysieren aber nur sehr langsam und werden deshalb bei der Aushärtung nur unvollständig eingebaut, was zu Blasenbildung und mechanisch schwachen Polymeren führt. Zudem neigen die freigesetzten langkettigen Aldehyde wegen ungenügender Verträglichkeit mit dem ausgehärteten Polyurethan zu Weichmacher-Migration, was sich durch Ausschwitzen (Bleeding), Substratverfärbung oder Spannungsrissbildung im Substrat zeigen kann. In dieser Schrift ebenfalls offenbart sind Oxazolidine abgeleitet von Lilyaldehyd. Diese arylaliphatischen Oxazolidine sind zusammen mit Isocyanaten aber nur sehr eingeschränkt lagerstabil und verursachen starke und langanhaltende Geruchsimmissionen durch freigesetzten Lilyaldehyd, der ausserdem potentiell allergieauslösend ist.

### Darstellung der Erfindung

Aufgabe der vorliegenden Erfindung ist es daher, Oxazolidine bereitzustellen, welche geeignet sind als latente Härter für Polyurethane und die Nachteile des Standes der Technik überwinden.

Überraschenderweise wurde gefunden, dass eine Verbindung der Formel (I) wie in Anspruch 1 beschrieben diese Aufgabe löst. Die Verbindung der Formel (I) enthält mindestens eine gegebenenfalls substituierte Oxazolidino-Gruppe oder strukturverwandte Gruppen mit Sechs- statt Fünfringstruktur bzw. mit Schwefel oder tertiärem Amin anstelle des Sauerstoffs im Ring.

Die Verbindung der Formel (I) stellt Oxazolidin-Härter zur Verfügung, die im ganzen Bereich der typischen Anwendungstemperatur flüssig sind, auch bei längerer Lagerdauer von mehreren Monaten bis zu ein Jahr und mehr nicht kristallisieren und dabei überraschend niedrigviskos sind, zusammen mit allen Arten von Isocyanaten lagerfähig sind und bei der Hydrolyse ein geruchsfreies, unflüchtiges und farbloses Blockierungsmittel freisetzen. Sie lassen sich somit einfach handhaben und ohne Einschränkungen auch in Innenräumen und in hellfarbigen Produkten einsetzen.

Isocyanatgruppen-haltige Zusammensetzungen enthaltend solche Oxazolidin-Härter zeichnen sich durch eine gute Lagerstabilität aus, auch zusammen mit sehr reaktiven aromatischen Isocyanaten, und härten mit Feuchtigkeit unter Gewährleistung einer ausreichenden Offenzeit schnell, vollständig und ohne Blasenbildung zu hochwertigen Polyurethanpolymeren. Dabei verursacht der freigesetzte Aldehyd keinerlei Geruchsimmissionen, wodurch solche Polyurethanzusammensetzungen auch für die Anwendung in Innenräumen und für grossflächige Anwendungen geeignet sind. Bei der Aushärtung entstehen hochwertige Polymere mit hoher mechanischer Festigkeit und Dehnbarkeit, einem moderaten Elastizitätsmodul und guter Hitzestabilität. Überraschenderweise zeigen die ausgehärteten Polymere keinerlei Neigung zu klebriger Oberfläche oder Substratschädigung aufgrund verstärkter Weichmachermigration, trotz des bei der Aushärtung freigesetzten, chemisch nicht in die Polyurethan-Matrix eingebauten Aldehyds der Formel (IV). Dies ist besonders überraschend, da dieser Aldehyd ein vergleichsweise hohes Molekulargewicht aufweist und entsprechend zu einem vergleichsweise hohen Gewichtsanteil im Polymer enthalten ist, und da er einen langkettigen, ausgesprochen hydrophoben Rest als Substituenten trägt, was eine eher schlechte Verträglichkeit mit dem hydrophilen, Wasserstoffbrücken aufweisenden Polymergerüst von Polyurethanen und damit eine starke Neigung zu Weichmachermigration erwarten liesse.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Verbindung der Formel (I), 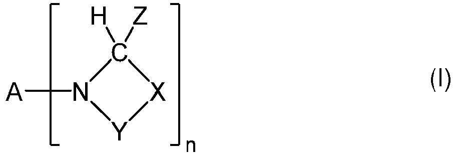 wobei
n für 1, 2 oder 3 steht,
Z für einen mit einer Alkyl- oder Alkoxy-Gruppe substituierten Aryl-Rest mit insgesamt 12 bis 26 C-Atomen steht,
A für einen n-wertigen organischen Rest mit einem Molekulargewicht im Bereich von 15 bis 10'000 g/mol steht,
X für O oder S oder NR⁰ steht, wobei R⁰ für einen einwertigen organischen Rest mit 1 bis 18 C-Atomen steht, und
Y für einen 1,2-Ethylen- oder 1,3-Propylen-Rest, welcher gegebenenfalls substituiert ist, steht,
wobei A und Y im Fall von n = 1 auch zu einem dreiwertigen Rest mit 4 bis 10 C-Atomen verbunden sein können.

Als "Silangruppe" wird eine an einen organischen Rest gebundene Silylgruppe mit einem bis drei, insbesondere zwei oder drei, Alkoxy-Resten am SiliciumAtom bezeichnet.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird üblicherweise mittels Gelpermeationschromatographie (GPC) gegen Polystyrol als Standard bestimmt.

Eine gestrichelte Linie in den Formeln stellt jeweils die Bindung zwischen einem Substituenten und dem zugehörigen Molekülrest dar.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "primäres Diamin" wird eine Verbindung mit zwei primären Aminogruppen bezeichnet.

Als "aromatisches Isocyanat" wird ein Isocyanat bezeichnet, dessen Isocyanatgruppen direkt an ein aromatisches C-Atom gebunden sind. Dementsprechend werden solche Isocyanatgruppen als "aromatische Isocyanatgruppen" bezeichnet.

Als "aliphatisches Isocyanat" wird ein Isocyanat bezeichnet, dessen Isocyanatgruppen direkt an ein aliphatisches C-Atom gebunden sind. Dementsprechend werden solche Isocyanatgruppen als "aliphatische Isocyanatgruppen" bezeichnet.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "Raumtemperatur" wird eine Temperatur von 23°C bezeichnet.

Als "Weichmacher" werden flüssige oder gelöste Substanzen bezeichnet, welche in einem ausgehärteten Polymer nicht chemisch eingebunden sind und typischerweise eine weichmachende Wirkung auf das Polymer ausüben.

Bevorzugt steht n für 1 oder 2. Diese Verbindungen der Formel (I) sind besonders einfach zugänglich und ermöglichen besonders lagerstabile einkomponentige Polyurethanzusammensetzungen.

Bevorzugt steht A für einen n-wertigen aliphatischen, cycloaliphatischen oder arylaliphatischen, gegebenenfalls Sauerstoff- oder Stickstoff- oder SiliciumAtome aufweisenden Kohlenwasserstoff-Rest mit einem Molekulargewicht im Bereich von 15 bis 6'000 g/mol.

Besonders bevorzugt steht A für einen n-wertigen aliphatischen, cycloaliphatischen oder arylaliphatischen Kohlenwasserstoff-Rest mit einem Molekulargewicht im Bereich von 15 bis 6'000 g/mol, der gegebenenfalls eine oder mehrere Gruppen ausgewählt aus Ether-, Ester-, Amino-, Aldimino-, Hydroxyl-, Amido-, Carbonat-, Urethan-, Harnstoff-, Nitril- und Silan-Gruppen aufweist.

A weist bevorzugt ein Molekulargewicht im Bereich von 15 bis 2'000 g/mol auf.

Insbesondere steht A für einen n-wertigen aliphatischen, cycloaliphatischen oder arylaliphatischen Kohlenwasserstoff-Rest mit einem Molekulargewicht im Bereich von 15 bis 2'000 g/mol, der gegebenenfalls eine oder mehrere Ether-, Ester-, Aldimino-, Hydroxyl-, Carbonat-, Urethan- oder Silan-Gruppen aufweist.

X steht bevorzugt für O oder NR⁰.

Besonders bevorzugt steht X für O.

R⁰ steht bevorzugt für einen Alkyl-, Cycloalkyl- oder Arylalkyl-Rest mit 1 bis 18 C-Atomen, der gegebenenfalls eine oder zwei Gruppen ausgewählt aus Carbonsäureester-, Nitril-, Nitro-, Phosphonsäureester-, Sulfon- und Sulfonsäureester-Gruppen trägt.

Besonders bevorzugt steht R⁰ für einen Alkyl-, Cycloalkyl- oder Arylalkyl-Rest mit 1 bis 12 C-Atomen steht, welcher gegebenenfalls eine oder zwei Carbonsäureester- oder Nitril-Gruppen trägt. Y steht bevorzugt für einen 1,2-Ethylen- oder 1,3-Propylen- oder 1,2-Propylen-Rest oder für einen gegebenenfalls substituierten und gegebenenfalls eine Silangruppe aufweisenden 3-Alkyloxy-1,2-propylen- oder 3-Aryloxy-1,2-propylen-Rest steht, oder kann im Fall von n = 1 und X = O auch für einen Rest der Formel 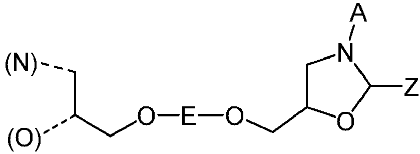 stehen, wobei E für einen zweiwertigen, gegebenenfalls eine oder mehrere Ether-, Ester- oder GlycidoxyGruppen tragenden Kohlenwasserstoff-Rest mit 6 bis 20 C-Atomen steht.

E steht bevorzugt für den Rest eines Glycidylethers nach Entfernung von zwei Glycidoxygruppen, insbesondere für 4,4'-Methylendiphenyl, 4,4'-(2,2-Propylen)diphenyl, 1,2- oder 1,3- oder 1,4-Phenyl, 1,4-Butylen, 1,6-Hexylen oder α,ω-Polyoxypropylen mit einem mittleren Molekulargewicht im Bereich von 96 bis 1'000 g/mol.

Y steht bevorzugt für einen 1,2-Ethylen- oder 1,2-Propylen- oder 3-Alkyloxy-1,2-propylen- oder 3-Aryloxy-1,2-propylen-Rest, oder für einen eine Silangruppe aufweisenden 3-Alkyloxy-1,2-propylen-Rest.

Besonders bevorzugt steht Y für 1,2-Ethylen oder 3-Alkyloxy-1,2-propylen- oder 3-Aryloxy-1,2-propylen.

Eine Verbindung der Formel (I), bei welcher X für O und Y für einen Rest, bei welchem N und O durch zwei C-Atome voneinander getrennt sind, stehen, ist ein Oxazolidin. Ein Oxazolidin ermöglicht Zusammensetzungen mit besonders schneller Aushärtung bei der Verwendung als latenter Härter.

Eine Verbindung der Formel (I), bei welcher X für O und Y für einen Rest, bei welchem N und O durch drei C-Atome voneinander getrennt sind, stehen, ist ein Oxazinan. Ein Oxazinan ermöglicht Zusammensetzungen mit besonders guter Lagerstabilität bei der Verwendung als latenter Härter.

Z steht bevorzugt für einen Rest der Formel (II), 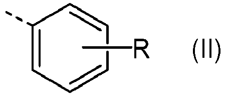 wobei R für einen Alkyl- oder Alkoxy-Rest mit 6 bis 20, bevorzugt 8 bis 16, C-Atomen steht.

R steht bevorzugt für einen verzweigten Rest. Eine solche Verbindung der Formel (I) ist besonders niedrigviskos.

R steht besonders bevorzugt für einen verzweigten Alkyl-Rest mit 10 bis 14 C-Atomen oder für einen verzweigten Alkoxy-Rest mit 8 bis 12 C-Atomen.

R steht insbesondere für einen verzweigten Alkyl-Rest mit 10 bis 14 C-Atomen. Ganz besonders bevorzugt steht R für einen Rest der Formel 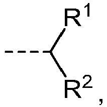 wobei R¹ und R² jeweils für einen Alkyl-Rest stehen und zusammen 9 bis 13 C-Atome aufweisen.

R steht bevorzugt in meta- oder para-Stellung, insbesondere in para-Stellung. Solche Reste Z sind besonders gut zugänglich.

Am meisten bevorzugt steht Z somit für einen Rest der Formel (II a), 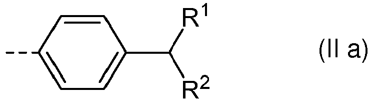 wobei R¹ und R² die genannten Bedeutungen aufweisen.

Die bevorzugten Reste Z sind besonders gut zugänglich und ermöglichen Verbindungen der Formel (I), welche bei Raumtemperatur typischerweise flüssig und besonders niedrigviskos sind.

Eine bevorzugte Ausführungsform der Erfindung ist eine Verbindung der Formel (I), bei welcher n für 1, Y für 1,2-Ethylen, X für O und A für A¹ steht, wobei A¹ für einen gegebenenfalls eine Hydroxylgruppe aufweisenden Alkyl-, Cycloalkyl- oder Aralkyl-Rest mit 1 bis 8 C-Atomen steht, insbesondere für Methyl, Ethyl, n-Propyl, Isopropyl, n-Butyl, Isobutyl, 2-Butyl, tert.Butyl, n-Hexyl, Isohexyl, 2-Ethylhexyl oder 2-Hydroxyethyl.

Eine solche Verbindung weist die Formel (I a) auf. Sie ist besonders geeignet als latenter Härter und/oder als Trocknungsmittel für Polyurethanzusammensetzungen. Für den Fall, dass der Rest A¹ eine Hydroxylgruppe aufweist, ist die Verbindung der Formel (I a) weiterhin geeignet als Edukt für die Herstellung von daraus aufgebauten Verbindungen der Formel (I) mit Ester-, Urethan- oder Carbonatgruppen.

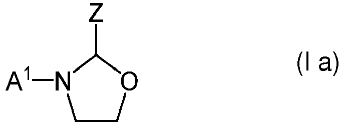

In der Formel (I a) weisen A¹ und Z die beschriebenen Bedeutungen auf. Besonders bevorzugt steht A¹ für Methyl, Ethyl, Propyl, Isopropyl oder n-Butyl.

Eine weitere bevorzugte Ausführungsform der Erfindung ist eine Verbindung der Formel (I), bei welcher n für 1, X für O und A für A² steht, wobei A² für einen eine Silangruppe aufweisenden Alkyl-Rest mit 1 bis 6 C-Atomen steht, insbesondere für 3-Trimethoxysilylpropyl, 3-Triethoxysilylpropyl oder 3-Dimethoxymethylsilyl propyl.

Eine solche Verbindung weist die Formel (I b) auf. Sie ist besonders geeignet als Haftvermittler für Polyurethanzusammensetzungen.

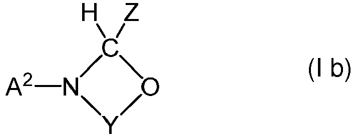

In der Formel (I b) weisen A², Y und Z die beschriebenen Bedeutungen auf.

Bevorzugte Verbindungen der Formel (I b) sind 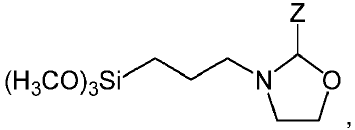 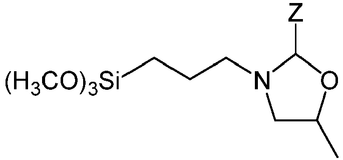 oder 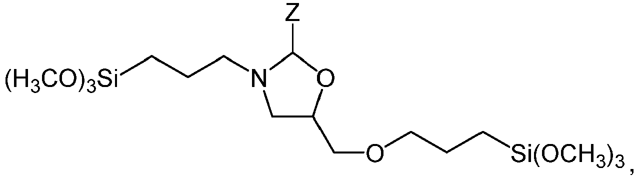 sowie entsprechende Verbindungen mit Ethoxygruppen anstelle der Methoxygruppen am Siliciumatom.

Eine weitere bevorzugte Ausführungsform der Erfindung ist eine Verbindung der Formel (I), bei welcher n für 1, X für O und A für Z-CH=N-A³--- steht, wobei A³ für einen Alkylen-, Cycloalkylen- oder Arylalkylen-Rest mit 1 bis 8 C-Atomen steht.

Eine solche Verbindung weist die Formel (I c) auf. Sie stellt insbesondere ein Aldimino-Oxazolidin dar und ist besonders geeignet als latenter Härter für Polyurethanzusammensetzungen.

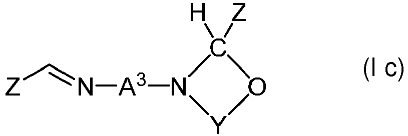

In der Formel (I c) weisen A³, Y und Z die beschriebenen Bedeutungen auf. Bevorzugt steht Y dabei für einen Ethylen- oder 3-Alkyloxy-1,2-propylen- oder 3-Aryloxy-1,2-propylen-Rest.

Besonders bevorzugte Verbindung der Formel (I c) sind 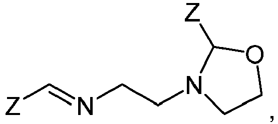 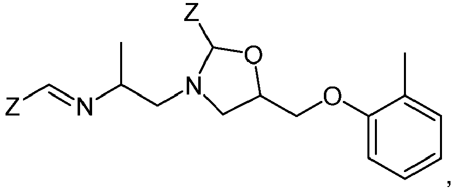 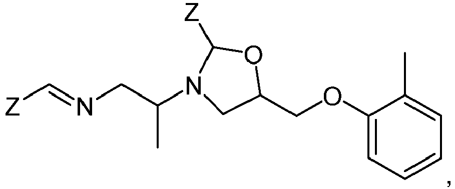 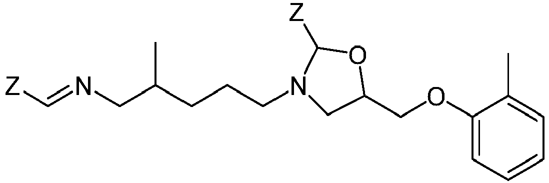 und

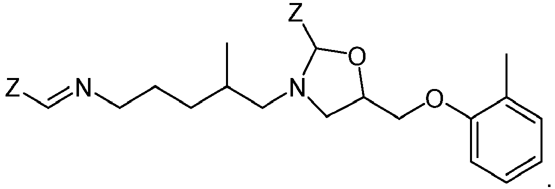

Eine weitere bevorzugte Ausführungsform der Erfindung ist eine Verbindung der Formel (I), bei welcher n für 2 und A für A⁴ steht, wobei A⁴ für einen gegebenenfalls eine oder mehrere Ether-, Ester-, Carbonat- oder Urethan-Gruppen tragenden Alkylen- oder Cycloalkylen- oder Arylalkylen-Rest mit 2 bis 50 C-Atomen steht.

Eine solche Verbindung weist die Formel (I d) auf. Bevorzugt steht X jeweils für O. Sie stellt somit insbesondere ein Bis-Oxazolidin dar und ist besonders geeignet als latenter Härter für Polyurethanzusammensetzungen.

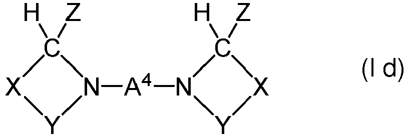

In der Formel (I d) weisen A⁴, X, Y und Z die bereits beschriebenen Bedeutungen auf.

Bevorzugt steht X für O.

Bevorzugt steht Y für 1,2-Ethylen, 1,2-Propylen oder 1,3-Propylen, insbesondere für 1,2-Ethylen.

Bevorzugt steht A⁴ für einen zwei Urethan-Gruppen tragenden Alkylen- oder Cycloalkylen- oder Arylalkylen-Rest mit 10 bis 20 C-Atomen.

Weiterhin bevorzugt steht A⁴ für einen eine Carbonat-Gruppe tragenden Alkylen-Rest mit 5 bis 7 C-Atomen.

Weiterhin bevorzugt steht A⁴ für einen gegebenenfalls eine oder mehrere Ether-Gruppen tragenden Alkylen- oder Cycloalkylen- oder Arylalkylen-Rest mit 2 bis 15 C-Atomen.

Weiterhin bevorzugt steht A⁴ für einen zwei Ester-Gruppen tragenden Alkylen- oder Cycloalkylen- oder Arylalkylen-Rest mit 10 bis 20 C-Atomen.

Besonders bevorzugte Verbindung der Formel (I d) sind 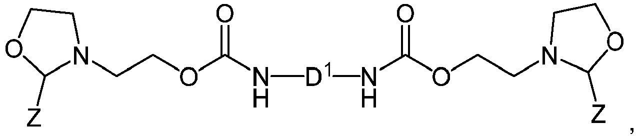 wobei D¹ für einen zweiwertigen Kohlenwasserstoff-Rest mit 6 bis 15 C-Atomen, insbesondere 1,6-Hexylen oder (1,5,5-Trimethylcyclohexan-1-yl)methan-1,3 oder 4(2)-Methyl-1,3-phenylen steht, 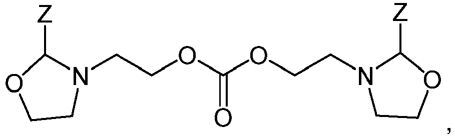 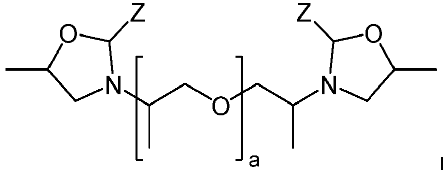 mit a = 2 oder 3, 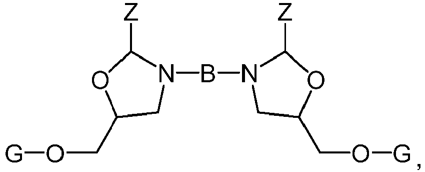 wobei B für einen gegebenenfalls Ether-Sauerstoff aufweisenden Alkylen- oder Cycloalkylen- oder Arylalkylen-Rest mit 2 bis 15 C-Atomen und G für einen gegebenenfalls Ether-Sauerstoff aufweisenden Kohlenwasserstoff-Rest mit 2 bis 25 C-Atomen steht, insbesondere für Phenyl, ein substituiertes Phenyl oder 2-Ethylhexyl, steht, 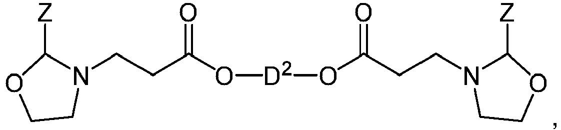 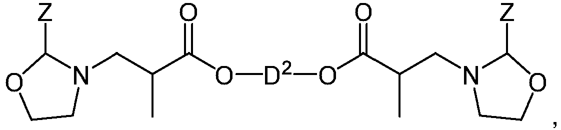 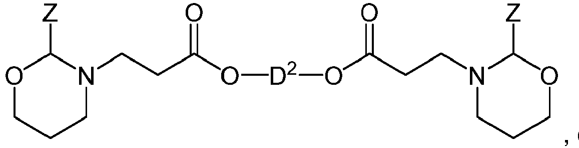 oder 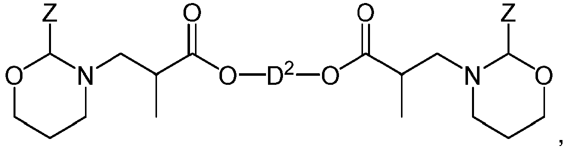 wobei D² für einen gegebenenfalls Ether-Sauerstoff aufweisenden Alkylen- oder Cycloalkylen- oder Arylalkylen-Rest mit 2 bis 15 C-Atomen steht.

Am meisten bevorzugte Verbindungen der Formel (I d) sind die Diurethane der Formel

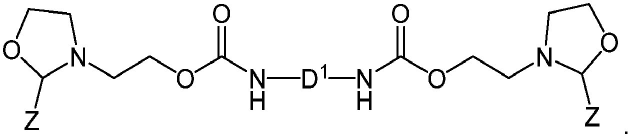

Eine weitere bevorzugte Ausführungsform der Erfindung ist eine Verbindung der Formel (I), bei welcher n für 1, X für O, A für A⁵ und Y für einen Rest der Formel 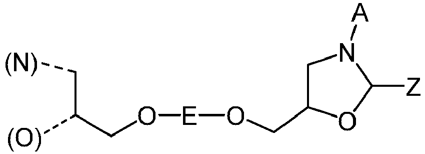 stehen, wobei A⁵ für einen gegebenenfalls eine oder mehrere Ether-Gruppen tragenden Alkyl-, Cycloalkyl- oder Aralkyl-Rest mit 1 bis 35 C-Atomen, insbesondere für Methyl, Ethyl, n-Propyl, Isopropyl, n-Butyl, Isobutyl, 2-Butyl, tert.Butyl, n-Hexyl, Isohexyl, 2-Ethylhexyl, Dodecyl oder einen Methoxy-terminierten Polyoxypropylen-Rest, welcher auch Oxyethylen-Anteile enthalten kann, steht.

Eine solche Verbindung weist die Formel (I e) auf. Sie stellt ein Bis-Oxazolidin dar und ist besonders geeignet als latenter Härter für Polyurethanzusammensetzungen.

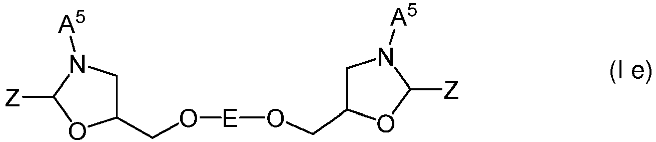

In der Formel (I e) weisen A⁵, E und Z die bereits beschriebenen Bedeutungen auf.

Eine weitere bevorzugte Ausführungsform der Erfindung ist eine Verbindung der Formel (I), bei welcher n für 1 steht, A und Y zu einem dreiwertigen, ein zusätzliches X und ein zusätzliches Z aufweisenden aliphatischen Kohlenwasserstoff-Rest mit 4 bis 10 C-Atomen verbunden sind und X für O steht.

Eine solche Verbindung weist insbesondere die Formel (I f) auf, 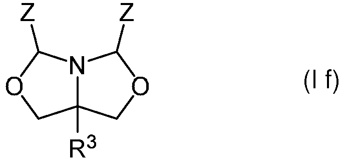 wobei R³ für einen Alkylrest mit 1 bis 4 C-Atomen steht, insbesondere für Methyl oder Ethyl, und Z die bereits genannten Bedeutungen aufweist.

Sie stellt ein anelliertes Bisoxazolidin dar und ist besonders geeignet als latenter Härter für Polyurethanzusammensetzungen.

Die Verbindung der Formel (I) wird bevorzugt erhalten aus der Umsetzung von mindestens einem Amin der Formel (III) mit mindestens einem Aldehyd der Formel (IV) in einer Kondensationsreaktion unter Entfernung von Wasser, 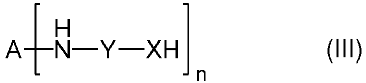 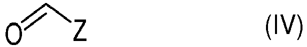 wobei A, X, n und Z die bereits genannten Bedeutungen aufweisen.

Für den Fall, dass A in Formel (I) eine Aldiminogruppe aufweist, steht bei A in Formel (III) an der entsprechenden Stelle insbesondere eine primäre Aminogruppe.

Der Aldehyd der Formel (IV) wird dabei stöchiometrisch oder insbesondere im stöchiometrischen Überschuss in Bezug auf die XH-Gruppen eingesetzt.

Bevorzugt wird die Umsetzung so geführt, dass
- das Amin der Formel (III) mit dem Aldehyd der Formel (IV) zu einer Reaktionsmischung vereint wird, wobei der Aldehyd stöchiometrisch oder insbesondere im stöchiometrischen Überschuss in Bezug auf die XH-Gruppen eingesetzt wird,
- und das Kondensationswasser und gegebenenfalls vorhandenes Lösemittel während oder nach der Vereinigung mit einer geeigneten Methode aus der Reaktionsmischung entfernt werden, gegebenenfalls unter deren Erwärmen und/oder Anlegen von Vakuum.

Bevorzugt wird das Kondensationswasser mittels Anlegen von Vakkum aus der erwärmten Reaktionsmischung entfernt, ohne dass dabei Lösemittel eingesetzt werden.

Eine Verbindung der Formel (I), bei welcher A eine oder mehrere Ester-, Carbonat- oder Urethan-Gruppen aufweist, kann insbesondere auch dadurch erhalten werden, dass eine eine Hydroxylgruppe aufweisende Verbindung der Formel (I) mit einem ein- oder mehrfunktionellen Carbonsäureester oder Carbonat oder Isocyanat umgesetzt wird.

Dabei wird als Hydroxylgruppen aufweisende Verbindung der Formel (I) insbesondere eine Verbindung der Formel (I a), bei welcher A¹ eine Hydroxylgruppe aufweist, eingesetzt. Am meisten bevorzugt wird dabei eine Verbindung der

Formel 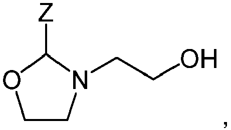 bei welcher Z die bereist beschriebenen Bedeutungen aufweist, eingesetzt.

Besonders bevorzugt ist die Umsetzung mit einem Carbonat oder einem handelsüblichen monomeren Diisocyanat wie insbesondere 1,6-Hexamethylendiisocyanat (HDI), 1-Isocyanato-3,3,5-trimethyl-5-isocyanatomethylcyclohexan (IPDI) oder 2,4- oder 2,6-Toluylendiisocyanat oder beliebige Gemische dieser Isomeren (TDI).

Eine Verbindung der Formel (I), bei welcher A eine oder mehrere Ester-, Amido- oder Nitril-Gruppen aufweist, ist auch dadurch erhältlich, dass eine Verbindung der Formel 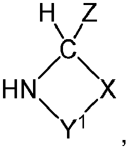 bei welcher Y¹ für einen 1,2-Ethylen- oder 1,3-Propylen- oder 1,2-Propylen-Rest steht und X und Z die bereits genannten Bedeutungen aufweisen, mit einem Michael-Akzeptor, insbesondere einem (Meth)acrylsäureester, Malein- oder Fumarsäurediester, Citraconsäurediester oder Itaconsäurediester, oder einem Amid dieser Ester, oder Acrylnitril, umgesetzt wird.

Dabei ist die Verbindung der Formel 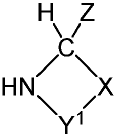 ihrerseits erhältlich aus der Umsetzung eines Amins der Formel H₂N-Y¹-XH mit dem Aldehyd der Formel (IV).

Geeignete Amine der Formel (III) für die Umsetzung mit dem Aldehyd der Formel (IV) zur Herstellung einer Verbindung der Formel (I) sind teilweise kommerziell verfügbar, oder sie lassen sich auf einfache Weise aus kommerziell verfügbaren Ausgangsstoffen herstellen.

Geeignet als Amin der Formel (III) ist ein kommerziell erhältliches aliphatisches Hydroxylamin, wie insbesondere N-Methylethanolamin, N-Ethylethanolamin, N-n-Propylethanolamin, N-Isopropylethanolamin, N-n-Butylethanolamin, N-Isobutylethanolamin, N-2-Butylethanolamin, N-tert.Butylethanolamin, N-n-Hexylethanolamin, N-Isohexylethanolamin oder N-(2-Ethylhexyl)ethanolamin.

Ein solches Amin der Formel (III) ist besonders geeignet für die Herstellung einer Hydroxylgruppen-freien Verbindung der Formel (I a).

Geeignet als Amin der Formel (III) ist weiterhin ein kommerziell erhältliches aliphatisches Dihydroxylamin, wie insbesondere Diethanolamin oder Diisopropanolamin.

Ein solches Amin der Formel (III) ist besonders geeignet für die Herstellung von eine Hydroxylgruppe aufweisenden Verbindungen der Formel (I a), welche anschliessend bevorzugt mit einem Carbonat oder Diisocyanat oder Ester zu einer Verbindung der Formel (I d) umgesetzt wird.

Geeignet als Amin der Formel (III) ist weiterhin ein Aminosilan wie insbesondere 3-Aminopropyltrimethoxysilan, 3-Aminopropyltriethoxysilan oder 3-Aminopropyldimethoxymethylsilan, welches mit einem Epoxid, wie insbesondere Ethylenoxid, Propylenoxid, 3-Glycidoxypropyltrimethoxysilan, 3-Glycidoxypropyltriethoxysilan, Phenylglycidylether, Kresylglycidylether oder ein Alkylglycidylether, umgesetzt ist. Dabei muss darauf geachtet werden, dass die bei der Umsetzung mit dem Epoxid gebildete Hydroxylgruppe möglichst nicht mit der Silangruppe reagiert. Eine baldige weitere Umsetzung mit dem Aldehyd der Formel (IV) ist also vorteilhaft. Bei der Umsetzung mit dem Aldehyd muss weiterhin beachtet werden, dass die Silangruppe möglichst nicht mit dem freigesetzten Wasser hydrolysiert. Ein schnelles Entfernen des Wasser und gegebenenfalls ein Mitverwenden von Trocknungsmitteln, wie beispielsweise Molekularsieb, ist bevorzugt.

Ein solches Amin der Formel (III) ist besonders geeignet für die Herstellung einer Verbindung der Formel (I b).

Geeignet als Amin der Formel (III) ist weiterhin ein Amin der Formel (III), bei welchem n für 1 steht und A eine primäre Aminogruppe aufweist, wie insbesondere N-(2-Aminoethyl)ethanolamin oder Umsetzungsprodukte von primären Diaminen mit Monoepoxiden im Molverhältnis 1:1. Besonders bevorzugt ist das Umsetzungsprodukt einer überstöchiometischen Menge von 1,2-Propandiamin oder 2-Methyl-1,5-pentandiamin mit Kresylglyciylether und nachfolgender Entfernung von nicht umgesetztem 1,2-Propandiamin bzw. 2-Methyl-1,5-pentan-diamin.

Ein solches Amin der Formel (III) ist besonders geeignet für die Herstellung einer Verbindung der Formel (I c), welche ein Aldimino-Oxazolidin darstellt.

Geeignet als Amin der Formel (III) ist weiterhin eine difunktionelle Verbindung, welche endständig je eine 2-Hydroxyethylamino- oder 2-Hydroxypropylamino-Gruppe aufweist.

Ein solches Amin ist insbesondere erhältlich durch Umsetzen eines handelsüblichen primären Diamins mit 2 mol Ethylenoxid oder Propylenoxid.

Ein solches Amin ist weiterhin erhältlich durch die Umsetzung eines handelsüblichen primären Diamins mit 2 mol eines handelsüblichen Monoglycidylethers, wie insbesondere Phenylglyciylether, Kresylglyciylether, tert.Butylphenylglyciylether, Cardanolglyciylether oder 2-Ethylhexylglyciylether.

Ein solches Amin ist weiterhin erhältlich durch die Umsetzung von 2 mol Ethanolamin oder Isopropanolamin oder 3-Amino-1-propanol mit einem handelsüblichen Diacrylat oder Dimethacrylat, wie insbesondere Butandioldiacrylat, Hexandioldiacrylat, Methylpentandioldiacrylat, Butandioldimethacrylat oder Hexandioldimethacrylat.

Diese Amine der Formel (III) sind besonders geeignet für die Herstellung einer Verbindung der Formel (I d).

Ebenfalls möglich ist die Umsetzung eines mehrfunktionellen Acrylats wie insbesondere Trimethylolpropantriacrylat mit Ethanolamin oder Isopropanolamin oder 3-Amino-1-propanol, wobei Verbindungen der Formel (I) mit n = 3 erhalten werden.

Geeignet als Amin der Formel (III) ist weiterhin ein Umsetzungsprodukt aus der Reaktion eines Monoamins, wie insbesondere Methylamin, Ethylamin, n-Propylamin, Isopropylamin, n-Butylamin, Isobutylamin, 2-Butylamin, tert.Butylamin, n-Hexylamin, Isohexylamin, 2-Ethylhexylamin, Dodecylamin, Benzylamin oder ein Methoxy-terminiertes Polyoxyalkylenamin wie insbesondere Jeffamine^{®} M-600 (von Huntsman), mit einem Diepoxid, insbesondere einem handelsüblichen Diglycidylether wie insbesondere Butandioldiglycidylether, Hexandioldiglycidylether, Dipropylenglycoldiglycidylether, Polypropylenglycoldiglyciylether, Bisphenol-A-Diglyciylether oder Bisphenol-F-Diglycidylether.

Ein solches Amin der Formel (III) ist besonders geeignet für die Herstellung einer Verbindung der Formel (I e).

Geeignet als Amin der Formel (III) ist weiterhin ein primäres Amin mit zwei Hydroxylgruppen, wie insbesondere 2-Amino-2-ethyl-1,3-propandiol. Dieses Amin ist insbesondere geeignet für die Herstellung einer Verbindung der Formel (I f), welche ein anelliertes Bisoxazolidin darstellt.

Geeignete handelsübliche primäre Diamine sind insbesondere 1,6-Hexandiamin, 2-Methyl-1,5-pentandiamin, 2,2(4),4-Trimethylhexamethylendiamin, 1-Amino-3-aminomethyl-3,5,5-trimethylcyclohexan, 4(2)-Methyl-1,3-cyclohexandiamin, 1,2-Cyclohexandiamin, Bis(4-aminocyclohexyl)methan, 1,3-Bis(aminomethyl)cyclohexan, 1,4-Bis(aminomethyl)cyclohexan, 1,3-Bis(aminomethyl)-benzol, Polyoxypropylendiamin mit einem mittleren Molekulargewicht im Bereich von 200 bis 2'000 g/mol, insbesondere 200 bis 500 g/mol, oder Trimethylolpropan- oder Glycerin-gestartetes Polyoxypropylentriamin mit einem mittleren Molekulargewicht im Bereich von 380 bis 500 g/mol.

Ein bevorzugter Aldehyd der Formel (IV) ist ein Aldehyd der Formel (IV a), wobei R die bereits beschriebenen Bedeutungen aufweist.

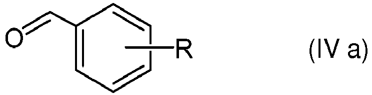

Ein besonders bevorzugter Aldehyd der Formel (IV) ist ein Aldehyd der Formel (IV b), wobei R¹ und R² die bereits beschriebenen Bedeutungen aufweisen.

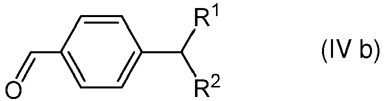

Als Aldehyd der Formel (IV) bevorzugt sind Benzaldehyde, welche in 3- oder 4-Stellung, insbesondere in 4-Stellung, einen verzweigten Alkyl- oder Alkoxy-Rest mit 6 bis 20, insbesondere 8 bis 16, C-Atomen tragen, insbesondere 4-Octylbenzaldehyd, 4-Nonylbenzaldehyd, 4-Decylbenzaldehyd, 4-Undecylbenzaldehyd, 4-Dodecylbenzaldehyd, 4-Tridecylbenzaldehyd, 4-Tetradecylbenzaldehyd, 4-Pentadecylbenzaldehyd, 4-Hexadecylbenzaldehyd, 4-Hexyloxybenzaldehyd, 4-Heptyloxybenzaldehyd, 4-Octyloxybenzaldehyd, 4-Nonyloxybenzaldehyd, 4-Decyloxybenzaldehyd, 4-Undecyloxybenzaldehyd, 4-Dodecyloxybenzaldehyd 4-Tridecyloxybenzaldehyd oder 4-Tetradecyloxybenzaldehyd, wobei die 4-Alkyl- bzw. Alkoxy-Reste jeweils verzweigt sind.

Als Aldehyd der Formel (IV) besonders bevorzugt sind Benzaldehyde, welche in 3- oder 4-Stellung, insbesondere in 4-Stellung, einen verzweigten C₁₀₋₁₄-Alkyl-Rest tragen, insbesondere 4-Decylbenzaldehyd, 4-Undecylbenzaldehyd, 4-Dodecylbenzaldehyd, 4-Tridecylbenzaldehyd oder 4-Tetradecylbenzaldehyd.

Als Aldehyd der Formel (IV) am meisten bevorzugt ist ein Gemisch enthaltend 4-Decylbenzaldehyde, 4-Undecylbenzaldehyde, 4-Dodecylbenzaldehyde, 4-Tridecylbenzaldehyde und 4-Tetradecylbenzaldehyde, deren Alkyl-Reste mehrheitlich verzweigt sind.

Der Aldehyd der Formel (IV) ist insbesondere erhältlich aus der Formylierung von mindestens einem Alkyl- und/oder Alkoxy-substituierten aromatischen Kohlenwasserstoff mit Kohlenmonoxid unter Einwirkung eines Säurekatalysators. Als Säurekatalysator eignet sich beispielsweise das System HCl-AlCl₃ (Gattermann-Koch-Reaktion).

In einem bevorzugten Herstellprozess wird die Formylierung mit HF-BF₃ als Säurekatalysator durchgeführt. Dies ist vorteilhaft, da dieses Verfahren besonders selektiv verläuft und der Aldehyd der Formel (IV) aus dem Reaktionsgemisch ohne Hydrolyseschritt abgetrennt und der Katalysator wiederverwendet werden kann, womit eine aufwendige Produktaufarbeitung und Abfallentsorgung entfällt.

Bevorzugt stellt die Verbindung der Formel (I) ein Gemisch dar aus Verbindungen der Formel (I), bei welchen Z jeweils für einen Rest der Formel (II) steht und R dabei ausgewählt ist aus Alkyl-Resten mit 6 bis 20 C-Atomen, welche mehrheitlich verzweigt sind. Besonders bevorzugt ist R dabei ausgewählt aus mehrheitlich verzweigten Decyl-, Undecyl-, Dodecyl-, Tridecyl- und Tetradecyl-Resten.

Ein weiterer Gegenstand der Erfindung ist somit ein Gemisch aus Verbindungen der Formel (I), bei welchen Z jeweils für einen Rest der Formel (II) steht und R dabei ausgewählt ist aus mehrheitlich verzweigten 4-Decyl-, 4-Undecyl-, 4-Dodecyl-, 4-Tridecyl- und 4-Tetradecyl-Resten.

Ein solches Gemisch ist technisch besonders einfach zugänglich.

Die Verbindung der Formel (I) zeichnet sich dadurch aus, dass sie als solche wie auch in hydrolysierter Form unflüchtig und vollständig geruchsfrei ist, somit zu keinerlei Immissionen führt und sich auch in Innenräumen und für grossflächige Anwendungen belastungsfrei für Verarbeiter und Nutzer einsetzen lässt, ohne dass dazu Schutzmassnahmen notwendig wären. Weiterhin ist die Verbindung der Formel (I) farblos und kann damit auch in hellfarbigen Produkten eingesetzt werden. Schliesslich ist sie typischerweise sowohl bei Raumtemperatur als auch bei Wintertemperaturen flüssig und kann somit auch bei ungeheizter Lagerung und Transport einfach und ohne Einsatz von Lösemitteln verarbeitet werden.

Ein weiterer Gegenstand der Erfindung ist die Verwendung von mindestens einer Verbindung der Formel (I) als latenter Härter für Isocyanatgruppen-haltige Zusammensetzungen.

Die Verbindung der Formel (I) weist vorteilhafte Eigenschaften für die beschriebene Verwendung auf. Sie lässt sich ausgezeichnet in Isocyanatgruppen-haltige Zusammensetzungen einmischen und zeigt kaum Neigung zu Separation. Sie löst in Abwesenheit von Wasser bzw. Feuchtigkeit kaum Vernetzungsreaktionen der Isocyanatgruppen aus und ermöglicht dadurch eine gute Lagerstabilität. Bei Zutritt von Feuchtigkeit reagiert sie unter Hydrolyse rasch mit vorhandenen Isocyanatgruppen, wobei dies weitgehend ohne konkurrierende Isocyanat-Hydrolyse und somit ohne Blasenbildung abläuft. Und schliesslich ist der bei der Aushärtung freigesetzte Aldehyd schwerflüchtig und verseifungsstabil, verursacht keine Geruchsbelastung und weist überraschenderweise eine ausgezeichnete Verträglichkeit mit dem ausgehärteten Polymer auf, schwitzt kaum aus und migriert kaum in die Substrate.

In einer Ausführungsform der Erfindung wird die Verbindung der Formel (I) verwendet als Trocknungsmittel für Zusammensetzungen, welche mit Feuchtigkeit aushärten, insbesondere Isocyanatgruppen-haltige Zusammensetzungen. Dafür wird die Verbindung der Formel (I) bei der Herstellung der Zusammensetzung mitverwendet und reagiert mit bei der Herstellung vorhandener Feuchtigkeit. Für die Verwendung als Trocknungsmittel geeignet sind insbesondere Verbindungen der Formel (I a), insbesondere solche, bei welchen der Rest A¹ frei ist von Hydroxylgruppen.

In einer weiteren Ausführungsform der Erfindung wird die Verbindung der Formel (I) verwendet als Haftvermittler für Zusammensetzungen, welche mit Feuchtigkeit aushärten, insbesondere Isocyanatgruppen-haltige Zusammensetzungen. Für diese Verwendung geeignet sind insbesondere Verbindungen der Formel (I b), welche mindestens eine Silangruppe aufweisen. Besonders geeignet sind Verbindungen der Formel (I b), bei welchen der Rest Y eine weitere Silangruppe aufweist. Solche Verbindungen der Formel (I b) werden insbesondere aus der Umsetzung von Aminosilanen mit Epoxysilanen und Aldehyden der Formel (IV) erhalten.

Ein weiterer Gegenstand der Erfindung ist eine Zusammensetzung umfassend
- mindestens eine Verbindung der Formel (I) und
- mindestens ein Polyisocyanat und/oder mindestens ein Isocyanatgruppen-haltiges Polyurethanpolymer.

Als Verbindung der Formel (I) geeignet sind die vorgängig beschriebenen. Besonders geeignet sind Verbindungen der Formel (I c), (I d), (I e) oder (I f). Am meisten geeignet sind Verbindungen der Formel (I c) oder (I d).

Als Polyisocyanat geeignet ist insbesondere ein im Handel erhältliches Polyisocyanat, insbesondere
- aromatische Di- oder Triisocyanate, bevorzugt 4,4'- oder 2,4'- oder 2,2'-Diphenylmethandiisocyanat oder beliebigen Gemischen dieser Isomeren (MDI), 2,4- oder 2,6-Toluylendiisocyanat oder beliebige Gemische dieser Isomeren (TDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Tris-(4-isocyanatophenyl)methan oder Tris-(4-isocyanatophenyl)thiophosphat; bevorzugt MDI oder TDI;
- aliphatische, cycloaliphatische oder arylaliphatische Di- oder Triisocyanate, bevorzugt 1,4-Tetramethylendiisocyanat, 2-Methylpentamethylen-1,5-diisocyanat, 1,6-Hexamethylendiisocyanat (HDI), 2,2,4- und/oder 2,4,4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- und/oder -2,6-diisocyanatocyclohexan (H₆TDI), 1-Isocyanato-3,3,5-trimethyl-5-isocyanatomethylcyclohexan (IPDI), Perhydro-2,4'- und/oder -4,4'-diphenylmethandiisocyanat (H₁₂MDI), 1,3- oder 1,4-Bis-(isocyanatomethyl)cyclohexan, m- oder p-Xylylendiisocyanat, Tetramethyl-1,3- oder -1,4-xylylendiisocyanat, 1,3,5-Tris-(isocyanatomethyl)benzol, Bis-(1-Isocyanato-1-methylethyl)naphthalin, Dimer- oder Trimerfettsäureisocyanate wie insbesondere 3,6-Bis-(9-isocyanatononyl)-4,5-di(1-heptenyl)cyclohexen (Dimeryldiisocyanat); bevorzugt H₁₂MDI oder HDI oder IPDI;.
- Oligomere oder Derivate der genannten Di- oder Triisocyanate, insbesondere abgeleitet von HDI, IPDI, MDI oder TDI, insbesondere Oligomere enthaltend Uretdion- oder Isocyanurat- oder Iminooxadiazindion-Gruppen oder verschiedene dieser Gruppen; oder zwei- oder mehrwertige Derivate enthaltend Ester- oder Harnstoff- oder Urethan- oder Biuret- oder Allophanat- oder Carbodiimid- oder Uretonimin- oder Oxadiazintrion-Gruppen oder verschiedene dieser Gruppen. Solche Polyisocyanate stellen in der Praxis üblicherweise Gemische von Substanzen mit unterschiedlichen Oligomerisierungsgraden und/oder chemischen Strukturen dar. Insbesondere weisen sie eine mittlere NCO-Funktionalität von 2.1 bis 4.0 auf.

Als Polyisocyanat bevorzugt sind aliphatische, cycloaliphatische oder aromatische Diisocyanate, insbesondere HDI, TMDI, Cyclohexan-1,3- oder -1,4-diisocyanat, IPDI, H₁₂MDI, 1,3- oder 1,4-Bis-(isocyanatomethyl)cyclohexan, XDI, TDI, MDI, 1,3- oder 1,4-Phenylendiisocyanat oder Naphthalin-1,5-diisocyanat (NDI).

Ein besonders bevorzugtes Polyisocyanat ist HDI, IPDI, H₁₂MDI, TDI, MDI oder eine bei Raumtemperatur flüssige Form von MDI, insbesondere HDI, IPDI, TDI oder MDI.

Eine bei Raumtemperatur flüssige Form von MDI stellt entweder durch partielle chemische Modifizierung - insbesondere Carbodiimidisierung bzw. Uretoniminbildung oder Adduktbildung mit Polyolen - verflüssigtes 4,4'-MDI dar, oder es ist ein durch Abmischen gezielt herbeigeführtes oder durch den Herstellungsprozess bedingtes Gemisch von 4,4'-MDI mit anderen MDI-Isomeren (2,4'-MDI und/oder 2,2'-MDI), und/oder MDI-Oligomeren und/oder MDI-Homologen (PMDI).

Am meisten bevorzugt ist IPDI, TDI oder MDI.

Ein geeignetes Isocyanatgruppen-haltiges Polyurethanpolymer wird insbesondere erhalten aus der Umsetzung von mindestens einem Polyol mit einer überstöchiometrischen Menge von mindestens einem Polyisocyanat. Die Umsetzung wird bevorzugt unter Feuchtigkeitsausschluss bei einer Temperatur im Bereich von 50 bis 160 °C durchgeführt, gegebenenfalls in Anwesenheit geeigneter Katalysatoren. Das NCO/OH-Verhältnis liegt bevorzugt im Bereich von 1.3/1 bis 5/1, bevorzugt 1.5/1 bis 4/1, insbesondere 1.8/1 bis 3/1. Das nach der Umsetzung der OH-Gruppen im Reaktionsgemisch verbleibende Polyisocyanat, insbesondere monomeres Diisocyanat, kann entfernt werden, insbesondere mittels Destillation, was im Fall eines hohen NCO/OH-Verhältnisses bevorzugt ist. Das erhaltene Polyurethanpolymer weist bevorzugt einen Gehalt an freien Isocyanatgruppen im Bereich von 1 bis 10 Gewichts-%, insbesondere 1.5 bis 6 Gewichts-%, auf. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Als Polyisocyanat zur Herstellung eines Isocyanatgruppen-haltigen Polyurethanpolymers bevorzugt sind die bereits genannten Polyisocyanate, insbesondere die Diisocyanate, bevorzugt MDI, TDI, IPDI, HDI oder H₁₂MDI, insbesondere MDI, TDI, IPDI oder HDI.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder mehreren aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD). Bevorzugte Polyetherpolyole sind Polyoxypropylendiole oder Polyoxypropylentriole, oder sogenannte Ethylenoxid-terminierte (EO-endcapped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch schliesslich primäre Hydroxylgruppen aufweisen. Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure oder Hexahydrophthalsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/ Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; sowie hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Am meisten bevorzugt sind Polyetherpolyole, insbesondere Polyoxypropylendi- oder -triole oder Ethylenoxid-terminierte Polyoxypropylendi- oder -triole. Bevorzugt sind Polyole mit einem mittleren Molekulargewicht im Bereich von 400 bis 20'000 g/mol, bevorzugt von 1'000 bis 10'000 g/mol.

Bevorzugt sind Polyole mit einer mittleren OH-Funktionalität im Bereich von 1.6 bis 3.

Bevorzugt sind bei Raumtemperatur flüssige Polyole.

Für die Herstellung eines bei Raumtemperatur festen Polyurethanpolymers sind bei Raumtemperatur feste Polyole bevorzugt.

Bei der Herstellung eines Isocyanatgruppen-haltigen Polyurethanpolymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Bevorzugt ist die Mitverwendung von 1,4-Butandiol für Anwendungen, bei welchen ein besonders hohe Festigkeiten gewünscht sind.

Das Isocyanatgruppen-haltige Polyurethanpolymer weist bevorzugt ein mittleres Molekulargewicht im Bereich von 1'000 bis 20'000 g/mol, insbesondere 1'500 bis 10'000 g/mol, auf.

Bevorzugt ist es bei Raumtemperatur flüssig.

Für eine Verwendung in einem Heissschmelzklebstoff ist ein bei Raumtemperatur festes Polyurethanpolymer bevorzugt, welches ausgehend von mindestens einem bei Raumtemperatur festen Polyol hergestellt ist. Ein geeignetes bei Raumtemperatur festes Polyol ist bei Raumtemperatur kristallin, teilkristallin oder amorph. Bevorzugt liegt sein Schmelzpunkt im Bereich von 50 bis 180 °C, insbesondere 70 bis 150 °C. Bevorzugt sind Polyesterpolyole, insbesondere solche abgeleitet von Hexandiol und Adipinsäure oder Dodecandicarbonsäure, oder Acrylatpolyole. Das Polyurethanpolymer wird insbesondere bei einer Temperatur oberhalb des Schmelzpunktes des bei Raumtemperatur festen Polymers hergestellt.

Bevorzugt enthält die Zusammensetzung mindestens ein Isocyanatgruppen-haltiges Polyurethanpolymer.

Zusätzlich zu einem Isocyanatgruppen-haltigen Polyurethanpolymer kann die Zusammensetzung weiterhin mindestens ein Diisocyanat und/oder ein Oligomer oder Polymer eines Diisocyanates enthalten, insbesondere ein IPDI-Isocyanurat oder ein TDI-Oligomer oder ein gemischtes Isocyanurat auf Basis TDI/HDI oder ein HDI-Oligomer oder eine bei Raumtemperatur flüssige Form von MDI.

Bevorzugt enthält die Zusammensetzung neben mindestens einer Verbindung der Formel (I) und mindestens einem Polyisocyanat und/oder Isocyanatgruppen-haltigen Polyurethanpolymer zusätzlich einen oder mehrere weitere Bestandteile, welche insbesondere ausgewählt sind aus Katalysatoren, Füllstoffen, Weichmachern und Lösemitteln.

Geeignete Katalysatoren sind insbesondere Katalysatoren für die Hydrolyse von Oxazolidinogruppen, insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignete Katalysatoren sind weiterhin Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignet sind insbesondere auch Kombinationen von verschiedenen Katalysatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, Kaoline, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate, insbesondere hydriertes Diisononylphthalat bzw. Diisononylcyclohexan-1,2-dicarboxylat (DINCH), Terephthalate, insbesondere Dioctylterephthalat, Trimellitate, Adipate, insbesondere Dioctyladipat, Azelate, Sebacate, Benzoate, Glykolether, Glykolester, organische Phosphor- oder Sulfonsäureester, Polybutene, Polyisobutene oder von natürlichen Fetten oder Ölen abgeleitete Weichmacher, insbesondere epoxidiertes Soja- oder Leinöl.

Geeignete Lösemittel sind insbesondere Aceton, Methylethylketon, Methyl-n-propylketon, Diisobutylketon, Methylisobutylketon, Methyl-n-amylketon, Methylisoamylketon, Acetylaceton, Mesityloxid, Cyclohexanon, Methylcyclohexanon, Ethylacetat, Propylacetat, Butylacetat, n-Butylpropionat, Diethylmalonat, 1-Methoxy-2-propylacetat, Ethyl-3-ethoxypropionat, Diisopropylether, Diethylether, Dibutylether, Diethylenglykoldiethylether, Ethylenglykoldiethylether, Ethylenglykolmonopropylether, Ethylenglykolmono-2-ethylhexylether, Acetale wie insbesondere Methylal, Ethylal, Propylal, Butylal, 2-Ethylhexylal, Dioxolan, Glycerolformal oder 2,5,7,10-Tetraoxaundecan (TOU), sowie Toluol, Xylol, Heptan, Octan, Naphtha, White Spirit, Petrolether oder Benzin, insbesondere Solvesso^{™}-Typen (von Exxon), weiterhin Methylenchlorid, Propylencarbonat, Butyrolacton, N-Methylpyrrolidon oder N-Ethylpyrrolidon.

Die Zusammensetzung kann weitere für Polyurethanzusammensetzungen gebräuchliche Zusätze enthalten. Insbesondere können die folgenden Hilfs- und Zusatzstoffe vorhanden sein:
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Farbstoffe;
- Trocknungsmittel, insbesondere Molekularsiebpulver, Calciumoxid, hochreaktive Isocyanate wie p-Tosylisocyanat, monomere Diisocyanate oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- latente Härter oder Vernetzer, insbesondere Aldimine, Ketimine, Enamine oder nicht der Formel (I) entsprechende Oxazolidine;
- weitere Katalysatoren, welche die Reaktion der Isocyanatgruppen beschleunigen, insbesondere Salze, Seifen oder Komplexe von Zinn, Zink, Bismut, Eisen, Aluminium, Molybdän, Dioxomolybdän, Titan, Zirkonium oder Kalium, insbesondere Zinn(II)-2-ethylhexanoat, Zinn(II)-neodecanoat, Zink(II)-acetat, Zink(II)-2-ethylhexanoat, Zink(II)-laurat, Zink(II)-acetylacetonat, Aluminiumlactat, Aluminiumoleat, Diisopropoxytitan-bis-(ethylacetoacetat) oder Kaliumacetat; tertiäre Aminogruppen enthaltende Verbindungen, insbesondere N-Ethyldiisopropylamin, N,N,N',N'-Tetramethylalkylendiamine, Pentamethylalkylentriamine und höhere Homologe davon, Bis-(N,N-diethylaminoethyl)-adipat, Tris(3-dimethylaminopropyl)amin, 1,4-Diazabicyclo[2.2.2]octan (DABCO), 1,8-Diazabicyclo[5.4.0]undec-7-en (DBU), 1,5-Diazabicyclo[4.3.0]-non-5-en (DBN), N-Alkylmorpholine, N,N'-Dimethylpiperazin; stickstoffaromatische Verbindungen wie 4-Dimethylamino-pyridin, N-Methylimidazol, N-Vinylimidazol oder 1,2-Dimethylimidazol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- natürliche Harze, Fette oder Öle wie Kolophonium, Schellack, Leinöl, Rizinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, Stabilisatoren gegen Oxidation, Wärme, Licht oder UV-Strahlung oder Biozide;
oder weitere üblicherweise in feuchtigkeitshärtenden Zusammensetzungen eingesetzte Substanzen.

Es kann sinnvoll sein, gewisse Substanzen vor dem Einmischen in die Zusammensetzung chemisch oder physikalisch zu trocknen.

Insbesondere kann die Zusammensetzung neben mindestens einer Verbindung der Formel (I) weitere latente Härter enthalten, insbesondere Oxazolidine, welche nicht der Formel (I) entsprechen, oder Polyaldimine.

In einer bevorzugten Ausführungsform der Erfindung enthält die Zusammensetzung eine Kombination aus Verbindungen der Formel (I) und mindestens einem Polyaldimin.

Dabei ist das Polyaldimin insbesondere ein geruchsfreies Polyaldimin.

Bevorzugt weist das Polyaldimin die Formel (V) oder (VI) auf, 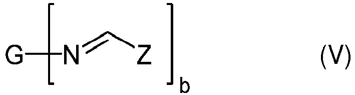 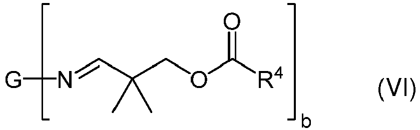 wobei
b für 2 oder 3 steht,
G für einen b-wertigen aliphatischen, cycloaliphatischen oder arylaliphatischen, gegebenenfalls Ether-Sauerstoff aufweisenden Kohlenwasserstoff-Rest mit einem Molekulargewicht im Bereich von 28 bis 6'000 g/mol steht,
R⁴ für einen Alkyl-Rest mit 7 bis 17 C-Atomen, insbesondere für einen linearen Alkyl-Rest mit 11 C-Atomen, steht,
und Z die bereits beschriebenen Bedeutungen aufweist.

Eine solche Zusammensetzung weist eine besonders gute Lagerstabilität auf und lässt sich optimal an die mechanischen Anforderungen für verschiedene Anwendungen anpassen. Die Zusammensetzung ist nach wie vor geruchsfrei und härtet bei Kontakt mit Feuchtigkeit.

Bei der Verwendung von Polyaldiminen der Formel (V) zeigt die Zusammensetzung eine besonders geringe Neigung zu Weichmachermigration.

Bei der Verwendung von Polyaldiminen der Formel (VI) zusätzlich zur Verbindung der Formel (I) zeigt die Zusammensetzung eine geringere Neigung zu Weichmachermigration, als wenn sie ohne Verbindung der Formel (I) mit einer entsprechend höheren Menge Polyaldimin der Formel (VI) ausgehärtet wurde.

Bevorzugt steht G für einen b-wertigen aliphatischen, cycloaliphatischen oder arylaliphatischen Kohlenwasserstoff-Rest mit einem Molekulargewicht im Bereich von 45 bis 500 g/mol.

Besonders bevorzugt ist G ausgewählt aus der Gruppe bestehend aus 1,6-Hexylen, (1,5,5-Trimethylcyclohexan-1-yl)methan-1,3, 4(2)-Methyl-1,3-cyclohexylen, 1,3-Cyclohexylen-bis(methylen), 1,4-Cyclohexylen-bis(methylen), 1,3-Phenylen-bis(methylen), 1,2-Cyclohexylen, Methylendicyclohexan-4-yl, α,ω-Polyoxypropylen mit einem mittleren Molekulargewicht im Bereich von 170 bis 470 g/mol und Trimethylolpropan- oder Glycerin-gestartetes Tri(ω-ροlyοxypropylen) mit einem mittleren Molekulargewicht im Bereich von 330 bis 450 g/mol.

In der Zusammensetzung liegt das Verhältnis zwischen den aus den vorhandenen latenten Härtern freisetzbaren Reaktivgruppen, also insbesondere primäre und sekundäre Aminogruppen, Hydroxylgruppen und Mercaptogruppen, und den Isocyanatgruppen bevorzugt im Bereich von 0.1 bis 1.9, besonders bevorzugt im Bereich von 0.2 bis 1.8.

Für eine Zusammensetzung, welche frei ist von Aldiminogruppen, liegt das Verhältnis bevorzugt im Bereich von 0.5 bis 1.8, insbesondere 0.8 bis 1.6.

Für eine Zusammensetzung, welche auch Aldiminogruppen enthält, liegt das Verhältnis bevorzugt im Bereich von 0.2 bis 1.5, insbesondere 0.5 bis 1.3.

Die Zusammensetzung enthält bevorzugt einen Gehalt an Polyisocyanaten und Isocyanatgruppen-haltigen Polyurethanpolymeren im Bereich von 5 bis 90 Gewichts-%, insbesondere 10 bis 80 Gewichts-%.

Die Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und stellt insbesondere ein Fass, ein Container, ein Hobbock, ein Eimer, ein Kanister, eine Büchse, ein Beutel, ein Schlauchbeutel, eine Kartusche oder eine Tube dar.

Die Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen und welche als solche lagerstabil ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Die Zusammensetzung ist bevorzugt einkomponentig. Sie ist bei geeigneter Verpackung und Aufbewahrung lagerstabil, typischerweise während mehreren Monaten bis zu einem Jahr oder länger.

Bei der Applikation der Zusammensetzung beginnt der Prozess der Aushärtung. Als Ergebnis davon entsteht die ausgehärtete Zusammensetzung.

Im Fall einer einkomponentigen Zusammensetzung wird diese als solche appliziert und beginnt darauf unter dem Einfluss von Feuchtigkeit bzw. Wasser auszuhärten. Zur Beschleunigung der Aushärtung kann der Zusammensetzung bei der Applikation eine Beschleuniger-Komponente, welche Wasser und/oder einen Katalysator enthält oder freisetzt, zugemischt werden, oder die Zusammensetzung kann nach ihrer Applikation mit einer solchen Beschleuniger-Komponente in Kontakt gebracht werden.

Im Fall einer zweikomponentigen Zusammensetzung wird diese nach dem Vermischen der beiden Komponenten appliziert und beginnt dabei durch interne Reaktion auszuhärten, wobei die Aushärtung gegebenenfalls durch die Einwirkung von externer Feuchtigkeit vervollständigt wird. Das Vermischen der beiden Komponenten kann kontinuiertlich oder batchweise mit dynamischen Mischern oder Statikmischern erfolgen.

Bei der Aushärtung reagieren die Isocyanatgruppen unter dem Einfluss von Feuchtigkeit mit den aus der Verbindung der Formel (I) freigesetzten Amino- und Hydroxyl- oder Mercapto-Gruppen und gegebenenfalls vorhandenen weiteren blockierten Aminogruppen. Ein Teil der Isocyanatgruppen, insbesondere die gegenüber den freigesetzten Reaktivgruppen überschüssigen, reagieren unter dem Einfluss von Feuchtigkeit untereinander und/oder mit gegebenenfalls in der Zusammensetzung vorhandenen weiteren Reaktivgruppen.

Die Gesamtheit dieser zur Aushärtung der Zusammensetzung führenden Reaktionen der Isocyanatgruppen wird auch als Vernetzung bezeichnet.

Die zur Aushärtung der einkomponentigen Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung ("Haut"). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die zur Vervollständigung der Aushärtung einer zweikomponentigen Zusammensetzung gegebenenfalls benötigte externe Feuchtigkeit stammt bevorzugt aus der Luft und/oder aus den Substraten.

Die Zusammensetzung wird bevorzugt bei Umgebungstemperatur appliziert, insbesondere im Bereich von etwa 0 bis 50°C, bevorzugt im Bereich von 5 bis 40°C.

Die Aushärtung der Zusammensetzung erfolgt bevorzugt ebenfalls bei Umgebungstemperatur.

Die Zusammensetzung verfügt über eine vergleichsweise lange Offenzeit.

Als "Offenzeit" wird die Zeitspanne bezeichnet, während der die Zusammensetzung verarbeitet oder nachbearbeitet werden kann, nachdem der Aushärtungsprozess begonnen hat.

Die Zeit bis zur Ausbildung einer Haut ("Hautbildungszeit") bzw. bis zur Klebefreiheit ("tack-free time") stellt dabei ein Mass für die Offenzeit dar.

Bei der Vernetzung wird ein Aldehyd der Formel (IV) freigesetzt. Dieser ist weitgehend unflüchtig und geruchlos und verbleibt zum grössten Teil in der ausgehärteten Zusammensetzung. Dort verhält er sich bzw. wirkt wie ein Weichmacher. Als solcher kann er grundsätzlich selber migrieren und/oder die Migration von weiteren Weichmachern beeinflussen. Der Aldehyd der Formel (IV) ist mit der ausgehärteten Zusammensetzung sehr gut verträglich, migriert selber kaum und löst auch keine verstärkte Migration von Weichmachern aus.

Bevorzugt stellt die Zusammensetzung einen Klebstoff oder einen Dichtstoff oder eine Beschichtung dar.

Bevorzugt ist der Klebstoff oder Dichtstoff oder die Beschichtung elastisch.

Als Klebstoff und/oder Dichtstoff ist die Zusammensetzung insbesondere geeignet für Klebe- und Abdichtungsanwendungen, insbesondere in der Bau- und Fertigungsindustrie oder im Fahrzeugbau, insbesondere für die Parkettverklebung, Anbauteilverklebung, Hohlraumversiegelung, Montage, Modulverklebung, Karosserieverklebung, Scheibenverklebung oder Fugenabdichtung. Elastische Verklebungen im Fahrzeugbau sind beispielsweise das Ankleben von Teilen wie Kunststoffabdeckungen, Zierleisten, Flansche, Stosstangen, Führerkabinen oder andere Anbauteile, an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Als Dichtstoff ist die Zusammensetzung insbesondere geeignet für das elastische Abdichten von Fugen, Nähten oder Hohlräumen aller Art, insbesondere von Fugen am Bau wie Dilatationsfugen oder Anschlussfugen zwischen Bauteilen. Insbesondere für das Abdichten von Dilatationsfugen an Bauwerken ist ein Dichtstoff mit weichelastischen Eigenschaften besonders geeignet.

Als Beschichtung ist die Zusammensetzung geeignet zum Schutz von Böden oder Mauern, insbesondere als Beschichtung von Balkonen, Terrassen, Plätzen, Brücken, Parkdecks, oder zur Abdichtung von Dächern, insbesondere Flachdächern oder schwach geneigten Dachflächen oder Dachgärten, oder im Innern von Gebäuden zur Wasserabdichtung, beispielsweise unter Fliesen oder Keramikplatten in Nasszellen oder Küchen, oder als Bodenbelag in Küchen, Industriehallen oder Fabrikationsräumen, oder als Abdichtung in Auffangwannen, Kanälen, Schächten oder Abwasserbehandlungsanlagen, oder zum Schutz von Oberflächen als Lack oder Versiegelung, oder als Vergussmasse zur Hohlraumversiegelung, als Nahtabdichtung oder als Schutzbeschichtung für beispielsweise Rohre.

Sie kann auch zu Reparaturzwecken als Abdichtung oder Beschichtung verwendet werden, beispielsweise von undichten Dachmembranen oder nicht mehr tauglichen Bodenbelägen oder insbesondere als Reparaturmasse für hochreaktive Spritzabdichtungen.

Die Zusammensetzung kann so formuliert sein, dass sie eine pastöse Konsistenz mit strukturviskosen Eigenschaften aufweist. Eine solche Zusammensetzung wird mittels einer geeigneten Vorrichtung appliziert, beispielsweise aus handelsüblichen Kartuschen oder Fässern oder Hobbocks, beispielsweise in Form einer Raupe, wobei diese eine im Wesentlichen runde oder dreieckige Querschnittsfläche aufweisen kann.

Die Zusammensetzung kann weiterhin so formuliert sein, dass sie flüssig und sogenannt selbstverlaufend oder nur leicht thixotrop ist und zur Applikation ausgegossen werden kann. Als Beschichtung kann sie beispielsweise anschliessend flächig bis zur erwünschten Schichtdicke verteilt werden, beispielsweise mittels einer Rolle, einem Schieber, einer Zahntraufel oder einer Spachtel. Dabei wird in einem Arbeitsgang typischerweise eine Schichtdicke im Bereich von 0.5 bis 3 mm, insbesondere 1.0 bis 2.5 mm, aufgetragen.

In einer Ausführungsform der Erfindung stellt die Zusammensetzung einen reaktiven Heissschmelzklebstoff dar. Dafür ist ein bei Raumtemperatur festes Polyurethanpolymer bevorzugt. Ein Heissschmelzklebstoff wird bevorzugt im geschmolzenen Zustand bei einer Temperatur im Bereich von 80 bis 180°C appliziert.

Als Verbindung der Formel (I) in einer Heissschmelzklebstoff-Zusammensetzung ist insbesondere eine Hydroxylgruppen aufweisende Verbindung der Formel (I a) geeignet, welche in einer solchen Menge eigesetzt wird, dass das Verhältnis zwischen der OH-Gruppe und den vorhandenen Isocyanatgruppen im Bereich von 0.05 bis 0.5 liegt. Auf diese Weise liegen nach der Umsetzung zwischen den OH-Gruppen und den Isocyanatgruppen noch genügend freie Isocyanatgruppen vor, um mit den aus der Hydrolyse der Verbindung der Formel (I) frei werdenen sekundären Aminogruppen und zumindest teilweise auch den frei werdenen OH-Gruppen unter Vernetzung zu reagieren.

Weiterhin ist eine Verbindung der Formel (I d) besonders geeignet in einer Heissschmelzklebstoff-Zusammensetzung, insbesondere ein Diurethan.

Die Verwendung einer Hydroxylgruppen-haltigen Verbindung der Formel (I) in einem reaktiven Heissschmelzklebstoff weist verschiedene Vorteile auf. Bei der Applikation des Klebstoffs im geschmolzenen Zustand, typischerweise bei Temperaturen im Bereich von 80 bis 180 °C, wird die Blasenbildung unterdrückt, wobei die Schwerflüchtigkeit und Geruchsfreiheit des freigesetzten Aldehyds bei diesen hohen Temperaturen ein enormer Vorteil darstellt, und bei der Verwendung einer Verbindung der Formel (I a) mit einer Hydroxylgruppe wird der Monomergehalt des verwendeten Polyurethanpolymers durch die Reaktion mit der OH-Gruppe reduziert.

In einer Ausführungsform der Erfindung stellt die Zusammensetzung einen Primer dar. Die Verbindungen der Formel (I) üben generell eine gute haftvermittelnde Wirkung aus, insbesondere da die Zusammensetzung nach der Aushärtung typischerweise freie Hydroxylgruppen enthält, die mit dem Substrat in Wechselwirkung treten können. Besonders bevorzugt enthält die Primer-Zusammensetzung mindestens eine Verbindung der Formel (I b), welche über ihre Silangruppe eine besonders gute haftvermittelnde Wirung ausübt.

Der Primer enthält bevorzugt mindestens ein Lösemittel. Optional enthält der Primer weitere Bestandteile wie insbesondere Katalysatoren, weitere Silane, Titanate oder Zirkonate, oder gegebenenfalls Pigmente, Füllstoffe, Netzmittel, Silangruppen aufweisende Polyurethanpolymere oder Epoxidharze.

Der Primer wird üblicherweise so appliziert, dass nach dem Verdampfen der Lösemittel ein geschlossener Film in einer Schichtdicke im Bereich von einigen Mikrometern bis zu einigen Hundert Mikrometern auf dem Substrat verbleibt. Er wird typischerweise dazu verwendet, die Haftung zwischen einem Substrat und einem Klebstoff und/oder Dichtstoff oder einer Beschichtung zu verbessern, indem der Primerfilm sowohl zum Substrat als auch zu der auf den Primerfilm aufgebrachten härtbaren Zusammensetzung Haftung aufbauen kann.

Der Primer wird typischerweise mit einem Pinsel oder einer Rolle in dünner Schicht auf eine Substratoberfläche aufgebracht. Nach einer geeigneten Wartezeit, während welcher das Lösemittel teilweise oder vollständig verdunstet, wird der Klebstoff oder Dichtstoff oder die Beschichtung auf die so vorbehandelte Oberfläche aufgetragen und weist typischerweise eine verbesserte Haftung auf.

Ein solcher Primer weist bevorzugt eine lange Offenzeit aus, d.h. übt seine haftvermittelnde Wirkung über einen langen Zeitraum nach der Applikation aus. Dadurch ist es beispielsweise möglich, ein zu verklebendes Bauteil mit vorappliziertem Primer ("vorgeprimert") zu vertreiben und dieses ohne Verlust der Haftkraft zu einem beliebigen späteren Zeitpunkt zu verkleben bzw. zu fügen. Auf diese Weise fällt der Primerauftrag und somit die Handhabung eines typischerweise lösemittelhaltigen, sicherheitstechnische Vorrichtungen wie Dampfabsaugung und Schutzkleidung voraussetzenden Produkts im Fügeprozess weg, was für viele Verarbeiter einen erheblichen Vorteil darstellt.

Geeignete Substrate, welche mit der Zusammensetzung verklebt oder abgedichtet oder beschichtet werden können, sind insbesondere
- Glas, Glaskeramik, Beton, Mörtel, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Aus der Applikation und Aushärtung der Zusammensetzung wird ein Artikel erhalten, welcher mit der Zusammensetzung verklebt oder abgedichtet oder beschichtet ist. Dieser Artikel kann ein Bauwerk oder ein Teil davon sein, insbesondere ein Bauwerk des Hoch- oder Tiefbaus, eine Brücke, ein Dach, ein Treppenhaus oder eine Fassade, oder er kann ein industrielles Gut oder ein Konsumgut sein, insbesondere ein Fenster, ein Rohr, ein Rotorblatt einer Windkraftanlage, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter, oder ein Anbauteil davon.

Die erfindungsgemässe Zusammensetzung weist vorteilhafte Eigenschaften auf. Sie ist unter Ausschluss von Feuchtigkeit lagerstabil, auch bei aromatischen Isocyanatgruppen. Sie verfügt über eine ausreichend lange Offenzeit, um über einen gewissen Zeitraum nach der Applikation einen nahtlosen Verlauf des applizierten Materials oder eine Positionierung oder Nachjustierung der damit verbundenen Objekte zu ermöglichen, was beispielsweise bei grossflächigen Beschichtungen, langen Dichtstreifen oder bei der Verklebung von grossen oder komplexen Objekten wichtig ist. Die Aushärtung erfolgt ohne störende Geruchsimmissionen schnell, zuverlässig und blasenfrei, sodass die Zusammensetzung ohne Einschränkung auch unter klimatisch ungünstigen Bedingungen wie hoher Luftfeuchtigkeit und/oder hoher Temperatur oder unter Einsatz von wasserhaltigen Beschleunigerkomponenten verwendbar ist. Bei der Aushärtung baut sie rasch Festigkeit auf, wobei die gebildete Haut auf der Oberfläche schon bald überraschend nicht-klebrig trocken ist, was insbesondere bei der Anwendung auf Baustellen sehr wertvoll ist, da dadurch Verschmutzungen durch beispielsweise Staub verhindert werden. Die Aushärtung verläuft vollständig und führt zu einem hochwertigen Material, welches eine hohe mechanische Festigkeit und Dehnbarkeit mit einem moderaten Elastizitätsmodul verbindet und sich somit auch für weichelastische Produkte eignet. Die ausgehärtete Zusammensetzung ist langlebig und insbesondere thermisch sehr beständig. Trotz ihres Gehalts an freigesetztem Aldehyd der Formel (IV) zeigt sie kaum durch Weichmachermigration ausgelöste Fehler wie Ausschwitzen ("Bleeding"), Verfärbung, Fleckenbildung, Erweichung oder Substratablösung und kann somit ohne Einschränkung auch auf porösen Substraten oder Spannungsriss-bildenden Kunststoffen und in Kombination mit Deckschichten eingesetzt werden.

Ein weiterer Gegenstand der Erfindung ist die Verwendung von mindestens einem Aldehyd der Formel (IV) 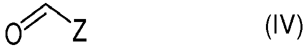 als Blockierungsmittel für Aminoalkohole, bei welchen das Stickstoff- und das Sauerstoff-Atom durch zwei oder drei C-Atome voneinander getrennt sind, wobei Z die bereits genannten Bedeutungen aufweist.

Bevorzugt wird dabei als Aldehyd der Formel (IV) ein Gemisch enthaltend 4-Decylbenzaldehyde, 4-Undecylbenzaldehyde, 4-Dodecylbenzaldehyde, 4-Tridecylbenzaldehyde und 4-Tetradecylbenzaldehyde, deren Alkyl-Reste mehrheitlich verzweigt sind, verwendet.

Bevorzugt werden die bereits genannten Aminoalkohole blockiert.

Aus dieser Verwendung werden Oxazolidine oder Oxazinane erhalten, welche über die bereits beschriebenen vorteilhaften Eigenschaften verfügen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

In den Beispielen nicht näher spezifizierte Chemikalien wurden bei Sigma-Aldrich bzw. Merck gekauft (in purum oder puriss.-Qualität) und wie erhalten eingesetzt.

Als "Normklima" ("NK") wird eine Temperatur von 23±1 °C und eine relative Luftfeuchtigkeit von 50±5% bezeichnet.

### Verwendete Aldehyde:

Aldehyd-1: Fraktioniertes Aldehydgemisch erhalten aus mittels HF-BF₃ katalysierter Formylierung von C₁₀₋₁₄-Alkylbenzol, enthaltend mehrheitlich verzweigte 4-(C₁₀₋₁₄-Alkyl)benzaldehyde. (mittleres Aldehyd-Equivalentgewicht 290 g/Eq)
Benzaldehyd (106.1 g/mol)
Isobutyraldehyd (72.1 g/mol)
2,2-Dimethyl-3-lauroyloxypropanal (284.4 g/mol)
Aldehyd-1 ist ein Gemisch von Aldehyden der Formel (IV).
Benzaldehyd und 2,2-Dimethyl-3-lauroyloxypropanal entsprechen nicht der Formel (IV).

### Herstellung von latenten Härtern:

Die **Aminzahl** (inklusive blockierte Aminogruppen) wurde bestimmt mittels Titration (mit 0.1 N HClO₄ in Essigsäure gegen Kristallviolett).

Die **Viskosität** wurde mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹ für Viskositäten < 150 Pa·s, Scherrate 1 s⁻¹ für Viskositäten > 150 Pa·s) gemessen.

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

### Oxazolidin A-1:

63.09 g Diethanolamin wurden in einem Rundkolben vorgelegt, mit 178.09 g Aldehyd-1 und 0.50 g Salicylsäure versetzt und die Reaktionsmischung bei 80°C und Vakuum gerührt, bis alles Wasser entfernt war. Es wurde eine gelbliche Flüssigkeit mit einer Aminzahl von 141.6 mg KOH/g erhalten.

FT-IR: 2954, 2923, 2871, 2857, 1704, 1607, 1509, 1465, 1425, 1378, 1343, 1298, 1213, 1174, 1045, 1018, 937, 884, 825, 722.

Das Oxazolidin **A-1** ist ein Mono-Oxazolidin und entspricht der Formel (I a).

### Oxazolidin A-2:

37.80 g Oxazolidin **A-1,** hergestellt wie vorgängig beschrieben, wurden unter Stickstoffatmosphäre in einem Rundkolben vorgelegt und aufgewärmt. Bei 80°C wurden tropfenweise 8.33 g 1,6-Hexamethylendiisocyanat zugegeben und dann bei 80°C gerührt, bis mittels IR-Spektroskopie keine Isocyanatgruppen mehr nachweisbar waren. Es wurde ein bei Raumtemperatur flüssiges, hochviskoses gelbes Öl mit einer Viskosität von 16.9 Pa·s bei 60°C und einer Aminzahl von 116.4 mg KOH/g erhalten. Nach einer Lagerzeit von 18 Monaten im verschlossenen Gebinde waren Konsistenz und Viskosität unverändert.

FT-IR: 2955, 2923, 2853, 1704, 1634, 1532, 1463, 1376, 1352, 1242, 1213, 1178, 1105, 1057, 1019, 825, 772, 722.

Das Oxazolidin **A-2** ist ein Bis-Oxazolidin und entspricht der Formel (I d).

### Oxazolidin A-3:

44.50 g Polyoxypropylen mit je einer endständigen 2-Hydroxypropylamino-Gruppe und einem mittleren Molekulargewicht von ca. 350 g/mol (Jeffamine^{®} C-346 von Huntsman) wurden in einem Rundkolben vorgelegt, mit 80.00 g Aldehyd-1 und 0.50 g Salicylsäure versetzt und die Reaktionsmischung bei 80 °C und Vakuum gerührt, bis alles Wasser entfernt war. Es wurde eine gelbliche Flüssigkeit mit einer Viskosität von 1.1 Pa·s bei 20°C und einer Aminzahl von 111.8 mg KOH/g erhalten.

FT-IR: 2957, 2923, 2870, 2853, 1726, 1704, 1645, 1606, 1575, 1456, 1375, 1304, 1212, 1194, 1168, 1094, 1062, 1017, 826, 722.

Das Oxazolidin **A-3** ist ein Bis-Oxazolidin und entspricht der Formel (I d).

### Oxazolidin A-4:

29.79 g N-(3-(2-Methylphenyloxy)-2-hydroxy-1-propyl)-1,2-propandiamin, hergestellt wie nachfolgend beschrieben, wurden in einem Rundkolben vorgelegt, mit 80.00 g Aldehyd-1 und 0.50 g Salicylsäure versetzt und die Reaktionsmischung bei 80 °C und Vakuum gerührt, bis alles Wasser entfernt war. Es wurde eine orangefarbene Flüssigkeit mit einer Viskosität von 3.0 Pa·s bei 20°C und einer Aminzahl von 116.5 mg KOH/g erhalten.

FT-IR: 2955, 2923, 2870, 2853, 1704, 1645, 1605, 1495, 1461, 1378, 1304, 1245, 1213, 1168, 1122, 1051, 1018, 826, 748, 722.

Das Oxazolidin **A-4** ist ein Imino-Oxazolidin und entspricht der Formel (I c).

N-(3-(2-Methylphenyloxy)-2-hydroxy-1-propyl)-1,2-propandiamin wurde hergestellt, indem 4.74 kg 1,2-Propandiamin unter Stickstoffatmosphäre vorgelegt, auf 70°C erwärmt und dann unter gutem Rühen langsam mit 2.93 kg o-Kresylglycidylether (Araldite^{®} DY-K, von Huntsman) versetzt wurde, wobei die Temperatur der Reaktionsmischung 70 bis 80°C betrug. Nach 1 Stunde bei 80°C wurde die Reaktionsmischung abgekühlt und 1,2-Propandiamin und weitere flüchtige Bestandteile destillativ mittels Dünnschichtverdampfer (0.5-1 mbar, Manteltemperatur 115°C) entfernt. Erhalten wurde eine leicht gelbliche Flüssigkeit mit einer Aminzahl von 478.7 mg KOH/g und einer Viskosität von 3.3 Pa·s bei 20°C.

### Oxazolidin A-5:

35.05 g N-(3-(2-Methylphenyloxy)-2-hydroxy-1-propyl)-2(4)-methyl-1,5-pentan-diamin, hergestellt wie nachfolgend beschrieben, wurden in einem Rundkolben vorgelegt, mit 80.00 g Aldehyd-1 und 0.50 g Salicylsäure versetzt und die Reaktionsmischung bei 80°C und Vakuum am Rotationsverdampfer gerührt, bis alles Wasser entfernt war. Es wurde eine orangefarbene Flüssigkeit mit einer Viskosität von 3.1 Pa·s bei 20°C und einer Aminzahl von 112.0 mg KOH/g erhalten.

FT-IR: 2955, 2923, 2870, 2853, 1704, 1647, 1605, 1495, 1461, 1377, 1304, 1245, 1168, 1122, 1051, 1038, 1018, 827, 748, 712.

Das Oxazolidin **A-5** ist ein Imino-Oxazolidin und entspricht der Formel (I c).

N-(3-(2-Methylphenyloxy)-2-hydroxy-1-propyl)-2(4)-methyl-1,5-pentandiamin wurde hergestellt, indem 4.65 kg 1,5-Diamino-2-methylpentan (Dytek^{®} A, von Invista) unter Stickstoffatmosphäre vorgelegt, auf 70°C erwärmt und dann unter gutem Rühren langsam mit 1.83 kg o-Kresylglycidylether (Araldite^{®} DY-K, von Huntsman) versetzt wurde, wobei die Temperatur der Reaktionsmischung 70 bis 80°C betrug. Nach 1 Stunde bei 80°C wurde die Reaktionsmischung abgekühlt und 1,5-Diamino-2-methylpentan und weitere flüchtige Bestandteile destillativ mittels Dünnschichtverdampfer (0.5-1 mbar, Manteltemperatur 160°C) entfernt. Erhalten wurde eine leicht gelbliche Flüssigkeit mit einer Aminzahl von 367.1 mg KOH/g und einer Viskosität von 6.5 Pa·s bei 20°C

### Oxazolidin A-6:

30.16 g Polyoxypropylendiamin mit einem mittleren Molekulargewicht von ca. 240 g/mol (Jeffamine^{®} D-230 von Huntsman) wurden in einem Rundkolben vorgelegt und auf 70°C aufgewärmt. Unter Rühren wurden langsam 37.55 g Phenylglycidylether zugegeben und dann bei 80°C während 2 Stunden unter Stickstoffatmosphäre gerührt, wobei ein Bis(α,β-hydroxylamin) entstand. Zu diesem Reaktionsprodukt wurden 80.00 g Aldehyd-1 und 0.50 g Salicylsäure gegeben und die Reaktionsmischung bei 80 °C und Vakuum gerührt, bis alles Wasser entfernt war. Es wurde eine gelbliche Flüssigkeit mit einer Viskosität von 3.5 Pa·s bei 20°C und einer Aminzahl von 100.9 mg KOH/g erhalten. FT-IR: 2956, 2923, 2870, 2853, 1704, 1645, 1600, 1587, 1496, 1456, 1376, 1301, 1244, 1170, 1101, 1077, 1042, 1018, 827, 752, 690.

Das Oxazolidin **A-6** ist ein Bis-Oxazolidin und entspricht der Formel (I d).

### Oxazolidin A-7:

Es wurde vorgegangen wie für Oxazolidin **A-6** beschrieben, aber anstelle des Polyoxypropylendiamins mit einem mittleren Molekulargewicht von ca. 240 g/mol wurden 250.00 g eines Polyoxypropylendiamins mit einem mittleren Molekulargewicht von ca. 2'000 g/mol (Jeffamine^{®} D-2000 von Huntsman) eingesetzt. Es wurde eine orangefarbene Flüssigkeit mit einer Viskosität von 1.8 Pa·s bei 20°C und einer Aminzahl von 31.3 mg KOH/g erhalten.

FT-IR: 2968, 2926, 2897, 2858, 1704, 1601, 1497, 1456, 1372, 1343, 1245, 1098, 1015, 925, 828, 753, 691.

Das Oxazolidin **A-7** ist ein Bis-Oxazolidin und entspricht der Formel (I d).

### Oxazolidin A-8:

16.97 g Methylpentandiol-Diacrylat (SR341, von Sartomer) wurden in einem Rundkolben vorgelegt, mit 9.16 g Ethanolamin versetzt, die Reaktionsmischung auf 50°C aufgewärmt und während 2 Stunden gerührt, wobei ein Bis-(α,β-hydroxylamin) entstand. Zu diesem Reaktionsprodukt wurden 45.83 g Aldehyd-1 und 0.50 g Salicylsäure gegeben und die Reaktionsmischung bei 80°C und Vakuum gerührt, bis alles Wasser entfernt war. Es wurde eine gelbliche Flüssigkeit mit einer Viskosität von 1.6 Pa·s bei 20°C und einer Aminzahl von 121.0 mg KOH/g erhalten.

FT-IR: 2955, 2923, 2871, 2853, 1734, 1704, 1647, 1607, 1458, 1378, 1301, 1212, 1179, 1109, 1054, 1018, 826, 722.

Das Oxazolidin **A-8** ist ein Bis-Oxazolidin und entspricht der Formel (I d).

### Oxazinan A-9:

Es wurde vorgegangen wie für Oxazolidin **A-8** beschrieben, aber anstelle von Ethanolamin wurden 11.27 g 3-Amino-1-propanol eingesetzt. Es wurde eine gelbliche Flüssigkeit mit einer Viskosität von 2.9 Pa·s bei 20 °C und einer Aminzahl von 118.6 mg KOH/g erhalten.

FT-IR: 2954, 2923, 2853, 1734, 1705, 1461, 1378, 1305, 1177, 1157, 1117, 1079, 1051, 977, 924, 826, 781, 722.

Das Oxazinan **A-9** ist ein Bis-Oxazinan und entspricht der Formel (I d).

### Oxazolidin A-10:

5.21 g N-(2-Aminoethyl)ethanolamin wurden in einem Rundkolben mit Wasserabscheider (Dean-Stark-Apparatur) vorgelegt, mit 30.56 g Aldehyd-1, 50 ml Cyclohexan und 0.10 g Salicylsäure versetzt und die Reaktionsmischung bei 120°C unter Rückfluss gekocht, bis alles Wasser abgeschieden war. Anschliessend wurde die Reaktionsmischung abgekühlt, filtriert und unter Vakuum am Rotationsverdampfer bei 80 °C die flüchtigen Bestandteile entfernt. Es wurde eine orangefarbene Flüssigkeit mit einer Viskosität von 1.1 Pa·s bei 20°C und einer Aminzahl von 160.2 mg KOH/g erhalten.

FT-IR: 2955, 2920, 2852, 1705, 1647, 1608, 1572, 1457, 1377, 1343, 1301, 1220, 1174, 1138, 1060, 1018, 986, 962, 826, 722.

Das Oxazolidin **A-10** ist ein Imino-Oxazolidin und entspricht der Formel (I c).

### Aminal A-11:

11.62 g N,N'-Diethylethylendiamin wurden in einem Rundkolben vorgelegt und unter Rühren mit 32.01 g Aldehyd-1 versetzt. Danach wurde die Reaktionsmischung bei 80°C und Vakuum am Rotationsverdampfer vom Reaktionswasser befreit. Es wurde eine orangefarbene Flüssigkeit mit einer Viskosität von 0.09 Pa·s bei 20°C und einer Aminzahl von 265.7 mg KOH/g erhalten.

FT-IR: 2957, 2924, 2871, 2853, 2792, 2525, 1706, 1607, 1508, 1465, 1423, 1377, 1340, 1280, 1226, 1164, 1055, 1019, 995, 958, 861, 824, 805, 722. Das Aminal **A-11** entspricht der Formel (I), wobei n für 1, X für NR⁰, R⁰ und A jeweils für Ethyl und Y für Ethylen stehen.

### Aminal A-12:

24.04 g N,N'-Dibenzylethylendiamin wurden in einem Rundkolben vorgelegt und unter Rühren mit 32.01 g Aldehyd-1 versetzt. Danach wurde die Reaktionsmischung bei 80°C und Vakuum am Rotationsverdampfer vom Reaktionswasser befreit. Es wurde eine orangefarbene Flüssigkeit mit einer Viskosität von 1.41 Pa·s bei 20°C und einer Aminzahl von 210.4 mg KOH/g erhalten.

FT-IR: 3085, 3062, 2954, 2923, 2870, 2852, 2792, 2722, 2520, 1804, 1705, 1605, 1507, 1453, 1423, 1377, 1338, 1295, 1248, 1212, 1153, 1120, 1072, 1028, 975, 911, 861, 827, 735, 690, 667.

Das Aminal **A-12** entspricht der Formel (I), wobei n für 1, X für NR⁰, R⁰ und A jeweils für Benzyl und Y für Ethylen stehen.

### Oxazolidin A-13:

11.72 g N-Butylethanolamin wurden in einem Rundkolben mit Wasserabscheider (Dean-Stark-Apparatur) vorgelegt, mit 29.68 g Aldehyd-1, 75 ml Cyclohexan und 0.10 g Salicylsäure versetzt und die Reaktionsmischung bei 120°C unter Rückfluss gekocht, bis alles Wasser abgeschieden war. Anschliessend wurde die Reaktionsmischung abgekühlt, filtriert und unter Vakuum am Rotationsverdampfer bei 80°C die flüchtigen Bestandteile entfernt. Es wurde eine gelbliche Flüssigkeit mit einer Viskosität von 0.07 Pa·s bei 20°C und einer Aminzahl von 142.1 mg KOH/g erhalten.

FT-IR: 2955, 2921, 2854, 2800, 1706, 1614, 1576, 1464, 1426, 1377, 1341, 1296, 1217, 1178, 1152, 1067, 1019, 957, 925, 826, 741, 722.

Das Oxazolidin **A-13** entspricht der Formel (I a), wobei A¹ für n-Butyl steht. Es ist insbesondere geeignet als Trocknungsmittel.

### Oxazolidin A-14:

13.92 g (50 mmol) 3-Glycidoxypropyltriethoxysilan (Dynasylan^{®} GLYEO, von Evonik) wurden in einem Rundkolben vorgelegt, mit 22.14 g (100 mmol) 3-Aminopropyltriethoxysilan (Dynasylan^{®} AMEO, von Evonik) versetzt und bei 70°C gerührt, bis die Epoxidgruppen umgesetzt waren. Anschliessend wurden 30.56 g Aldehyd-1 zugegeben und die Reaktionsmischung bei 80°C und Vakuum am Rotationsverdampfer vom Reaktionswasser befreit. Es wurde eine hellorange Flüssigkeit mit einer Viskosität von 0.35 Pa·s bei 20°C und einer Aminzahl von 87.6 mg KOH/g erhalten.

FT-IR: 2957, 2924, 2854, 1705, 1647, 1607, 1573, 1508, 1456, 1389, 1343, 1297, 1166, 1102, 1076, 1017, 954, 827, 774.

Das Oxazolidin **A-14** enthält ein Gemisch aus Oxazolidinosilan und Aldiminosilan. Das Oxazolidinosilan entspricht der Formel (I b), wobei A² für 3-Triethoxysilylpropyl und Y für einen in alpha-Stellung zum Oxazolidin-Sauerstoff mit 5-Triethoxysilyl-2-oxa-pentyl substituierten Ethylenrest steht. Es ist insbesondere geeignet als Haftvermittler.

### Oxazolidin B-1:

Es wurde vorgegangen wie für Oxazolidin **A-1** beschrieben, aber anstelle von Aldehyd-1 wurden 64.95 g Benzaldehyd eingesetzt. Es wurde eine hellbraune Flüssigkeit mit einer Aminzahl von 288.2 mg KOH/g erhalten.

### Oxazolidin B-2:

37.80 g Oxazolidin **B-1,** hergestellt wie vorgängig beschrieben, wurden unter Stickstoffatmosphäre in einem Rundkolben vorgelegt und aufgewärmt. Bei 80°C wurden tropfenweise 8.33 g 1,6-Hexamethylendiisocyanat zugegeben und dann bei 80°C gerührt, bis mittels IR-Spektroskopie keine Isocyanatgruppen mehr nachweisbar waren. Es wurde ein bei Raumtemperatur festes, gelbes Material mit einer Aminzahl von 181.4 mg KOH/g erhalten, welches bei 60°C eine Viskosität von 583.5 Pa·s aufwies.

### Oxazolidin B-3:

Es wurde vorgegangen wie für Oxazolidin **A-3** beschrieben, aber anstelle von Aldehyd-1 wurden 29.18 g Benzaldehyd eingesetzt. Es wurde eine hellbraune Flüssigkeit mit einer Viskosität von 1.9 Pa·s bei 20°C und einer Aminzahl von 219.4 mg KOH/g erhalten.

### Oxazolidin B-4:

Es wurde vorgegangen wie für Oxazolidin **A-4** beschrieben, aber anstelle von Aldehyd-1 wurden 29.18 g Benzaldehyd eingesetzt. Es wurde ein zähflüssiges dunkelorangefarbenes Öl mit einer Viskosität von 1'945 Pa·s bei 20°C und einer Aminzahl von 249.0 mg KOH/g erhalten, welches beim Stehenlassen bei Raumtemperatur während einigen Wochen langsam fest wurde.

### Oxazolidin B-5:

Es wurde vorgegangen wie für Oxazolidin **A-5** beschrieben, aber anstelle von Aldehyd-1 wurden 29.18 g Benzaldehyd eingesetzt. Es wurde ein orangefarbenes Öl mit einer Viskosität von 93.5 Pa·s bei 20°C und einer Aminzahl von 226.0 mg KOH/g erhalten.

### Oxazolidin B-6:

Es wurde vorgegangen wie für Oxazolidin **A-6** beschrieben, aber anstelle von Aldehyd-1 wurden 29.18 g Benzaldehyd eingesetzt. Es wurde ein orangefarbenes Öl mit einer Viskosität von 84.5 Pa·s bei 20°C und einer Aminzahl von 161.0 mg KOH/g erhalten.

### Oxazolidin B-7:

Es wurde vorgegangen wie für Oxazolidin **A-7** beschrieben, aber anstelle von Aldehyd-1 wurden 29.18 g Benzaldehyd eingesetzt. Es wurde eine orangefarbene Flüssigkeit mit einer Viskosität von 2.0 Pa·s bei 20°C und einer Aminzahl von 40.9 mg KOH/g erhalten.

### Oxazolidin B-8:

Es wurde vorgegangen wie für Oxazolidin **A-8** beschrieben, aber anstelle von Aldehyd-1 wurden 16.72 g Benzaldehyd eingesetzt. Es wurde eine gelbliche Flüssigkeit mit einer Viskosität von 2.0 Pa·s bei 20°C und einer Aminzahl von 216.6 mg KOH/g erhalten.

### Oxazinan B-9:

Es wurde vorgegangen wie für Oxazolidin **A-9** beschrieben, aber anstelle von Aldehyd-1 wurden 16.72 g Benzaldehyd eingesetzt. Es wurde eine gelbliche Flüssigkeit mit einer Viskosität von 10.5 Pa·s bei 20°C und einer Aminzahl von 205.1 mg KOH/g erhalten.

### Oxazolidin B-10:

15.00 g N-(2-Aminoethyl)ethanolamin wurden in einem Rundkolben mit Wasserabscheider (Dean-Stark-Apparatur) vorgelegt, mit 32.10 g Benzaldehyd, 50 ml Cyclohexan und 0.10 g Salicylsäure versetzt und die Reaktionsmischung bei 120°C unter Rückfluss gekocht, bis alles Wasser abgeschieden war. Anschliessend wurde die Reaktionsmischung abgekühlt, filtriert und unter Vakuum am Rotationsverdampfer bei 80°C die flüchtigen Bestandteile entfernt. Es wurde eine orangefarbene Flüssigkeit mit einer Viskosität von 0.83 Pa·s bei 20°C und einer Aminzahl von 385.1 mg KOH/g erhalten.

### Oxazolidin L-1:

Es wurde vorgegangen wie für Oxazolidin **A-1** beschrieben, aber anstelle von Aldehyd-1 wurden 174.65 g 2,2-Dimethyl-3-lauroyloxypropanal eingesetzt. Es wurde eine gelbliche Flüssigkeit mit einer Aminzahl von 141.2 mg KOH/g erhalten.

### Oxazolidin L-2:

39.73 g Oxazolidin **L-1,** hergestellt wie vorgängig beschrieben, wurden unter Stickstoffatmosphäre in einem Rundkolben vorgelegt und aufgewärmt. Bei 80°C wurden tropfenweise 8.33 g 1,6-Hexamethylendiisocyanat zugegeben und dann bei 80°C gerührt, bis mittels IR-Spektroskopie keine Isocyanatgruppen mehr nachweisbar waren. Es wurde eine gelbliche Flüssigkeit mit einer Viskosität von 6 Pa·s bei 20°C und einer Aminzahl von 115.2 mg KOH/g erhalten.

### Oxazolidin IB-2: (entspricht Incozol 4, von Incorez)

15.92 g N-(2-Hydroxyethyl)-2-isopropyloxazolidin (Incozol 3, von Incorez) wurden unter Stickstoffatmosphäre in einem Rundkolben vorgelegt und aufgewärmt. Bei 80°C wurden tropfenweise 8.33 g 1,6-Hexamethylendiisocyanat zugegeben und dann bei 80 °C gerührt, bis mittels IR-Spektroskopie keine Isocyanatgruppen mehr nachweisbar waren. Es wurde eine gelbliche Flüssigkeit mit einer Viskosität von 36 Pa·s bei 20°C und einer Aminzahl von 220.0 mg KOH/g erhalten, welche innerhalb von wenigen Wochen kristallisierte und für die weitere Verwendung aufgeschmolzen werden musste.

### Oxazolidin IB-10:

15.00 g N-(2-Aminoethyl)ethanolamin wurden in einem Rundkolben mit Wasserabscheider (Dean Stark Trap) vorgelegt, mit 21.81 g Isobutyraldehyd, 50 ml Cyclohexan und 0.10 g Salicylsäure versetzt und die Reaktionsmischung bei 120 °C unter Rückfluss gekocht, bis alles Wasser abgeschieden war. Anschliessend wurde die Reaktionsmischung abgekühlt, filtriert und unter Vakuum am Rotationsverdampfer bei 80 °C die flüchtigen Bestandteile entfernt. Es wurde eine gelbliche Flüssigkeit mit einer Viskosität von 0.01 Pa·s bei 20°C und einer Aminzahl von 521.7 mg KOH/g erhalten.

### Incozol 2:

Mono-Oxazolidin auf der Basis von N-Butylethanolamin und 2-Ethylhexanal, von Incorez.

Die Oxazolidine **A-1** bis **A-8, A-10, A-13** und **A-14,** das Oxazinan **A-9** und die Aminale **A-11** und **A-12** sind Verbindungen der Formel (I).

Die Oxazolidine **B-1** bis **B-8** und **B-10,** das Oxazinan **B-9** und die Oxazolidine **L-1, L-2, IB-2, IB-10** und **Incozol 2** entsprechen nicht der Formel (I) und dienen als Vergleich.

### Aldimin C-1:

50.00 g 2,2-Dimethyl-3-lauroyloxypropanal wurden in einem Rundkolben unter Stickstoffatmosphäre vorgelegt. Unter Rühren wurden 13.93 g 3-Aminomethyl-3,5,5-trimethylcyclohexylamin zugegeben und anschliessend die flüchtigen Bestandteile bei 80°C und 10 mbar Vakuum entfernt. Es wurde eine blassgelbe, geruchlose Flüssigkeit mit einer Viskosität von 0.2 Pa·s bei 20°C und einer Aminzahl von 153.0 mg KOH/g erhalten.

Das Aldimin **C-1** ist ein Dialdimin und entspricht der Formel (VI).

### Herstellung von Isocyanatgruppen-haltigen Polymeren

### Polymer P1:

400 g Polyoxypropylen-Diol (Acclaim^{®} 4200, von Covestro; OH-Zahl 28.5 mg KOH/g) und 52 g 4,4'-Diphenylmethandiisocyanat (Desmodur^{®} 44 MC L, von Covestro) wurden nach bekanntem Verfahren bei 80 °C zu einem bei Raumtemperatur flüssigen, NCO-terminierten Polyurethanpolymer mit einem Gehalt an freien Isocyanatgruppen von 1.85 Gewichts-% umgesetzt.

### Polymer P2:

590 g Polyoxypropylen-Diol (Acclaim^{®} 4200, von Covestro; OH-Zahl 28.5 mg KOH/g), 1180 g Polyoxypropylenpolyoxyethylen-Triol (Caradol^{®} MD34-02, von Shell; OH-Zahl 35.0 mg KOH/g) und 230 g Isophorondiisocyanat (Vestanat^{®} IPDI, von Evonik) wurden nach bekanntem Verfahren bei 80 °C zu einem bei Raumtemperatur flüssigen, NCO-terminierten Polyurethanpolymer mit einem Gehalt an freien Isocyanatgruppen von 2.10 Gewichts-% umgesetzt.

### Polymer P3:

356 g Polyoxypropylen-Diol (Acclaim^{®} 4200, von Covestro; OH-Zahl 28.5 mg KOH/g), 100 g Polyoxypropylen-Triol (Acclaim^{®} 6300, von Covestro; OH-Zahl 28.0 mg KOH/g) und 40 g einer Mischung aus 2,4- und 2,6-Toluylendiisocyanat im Verhältnis 80/20 (Desmodur^{®} T 80, von Covestro) wurden nach bekanntem Verfahren bei 80°C zu einem bei Raumtemperatur flüssigen, NCO-terminierten Polyurethanpolymer mit einem Gehalt an freien Isocyanatgruppen von 1.80 Gewichts-% umgesetzt

### Polymer P4:

300.0 g Polyoxypropylenpolyoxyethylen-Diol (Desmophen^{®} L300, von Covestro; OH-Zahl 190.0 mg KOH/g) und 228.8 g Isophorondiisocyanat (Vestanat^{®} IPDI, von Evonik) wurden nach bekanntem Verfahren bei 60°C zu einem bei Raumtemperatur flüssigen, NCO-terminierten Polyurethanpolymer mit einem Gehalt an freien Isocyanatgruppen von 7.91 Gewichts-% umgesetzt.

### Polymer P5:

1300 g Polyoxypropylen-Diol (Acclaim^{®} 4200, von Covestro; OH-Zahl 28.5 mg KOH/g), 2600 g Polyoxypropylen-Triol (Caradol^{®} MD34-02, von Shell; OH-Zahl 35.0 mg KOH/g), 600 g 4,4'-Diphenylmethandiisocyanat (Desmodur^{®} 44 MC L, von Covestro) und 500 g Diisodecylphthalat (Palatinol^{®} Z, BASF) wurden bei 80°C zu einem NCO-terminierten Polyurethanpolymeren mit einem Gehalt an freien Isocyanat-Gruppen von 2.05 Gewichts-% umgesetzt.

### Polyurethan-Zusammensetzungen (einkomponentig)

### Zusammensetzungen Z1 bis Z27:

Für jede Zusammensetzung wurden die in den Tabellen 1 bis 2 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Lagerstabilität wurden die **Viskosität (1d RT)** am Folgetag der Herstellung und die **Viskosität (7d 60°C)** nach einer Lagerung im verschlossenen Gebinde während 7 Tagen in einem 60°C warmen Umluftofen bestimmt, wie vorgängig beschrieben.

Als Mass für die Offenzeit wurde die Hautbildungszeit **(tack-free time)** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde jede Zusammensetzung auf eine PTFE-beschichtete Folie zu einem Film von 2 mm Dicke ausgegossen, während 7 Tagen im Normklima gelagert, einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung, E-Modul 5%** (bei 0.5-5% Dehnung) und **E-Modul 50%** (bei 0.5-50% Dehnung) geprüft.

Der **Aspekt** wurde visuell an den hergestellten Filmen beurteilt. Als "schön" wurde ein klarer Film mit nichtklebriger Oberfläche ohne Blasen bezeichnet. Der **Geruch** wurde durch Riechen mit der Nase im Abstand von 2 cm an den frisch hergestellten Filmen beurteilt. "Ja" bedeutet, dass ein Geruch deutlich wahrnehmbar war. "Nein" bedeutet, dass kein Geruch wahrnehmbar war.

Die Resultate sind in den Tabellen 1 bis 2 angegeben.

Bei den mit (Ref) gekennzeichneten Zusammensetzungen handelt es sich um Vergleichsbeispiele.

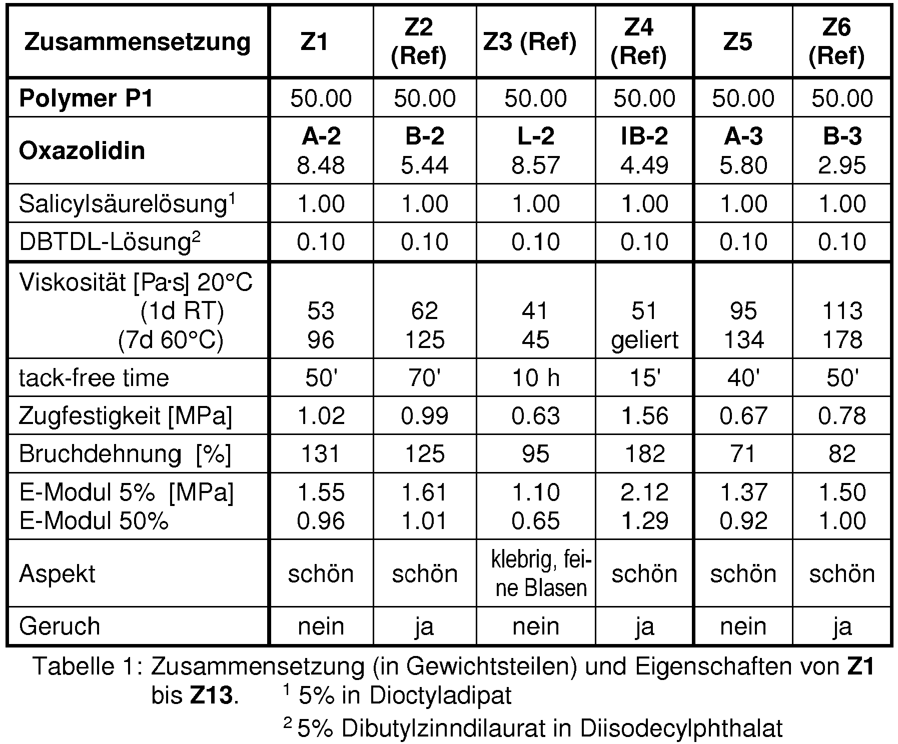

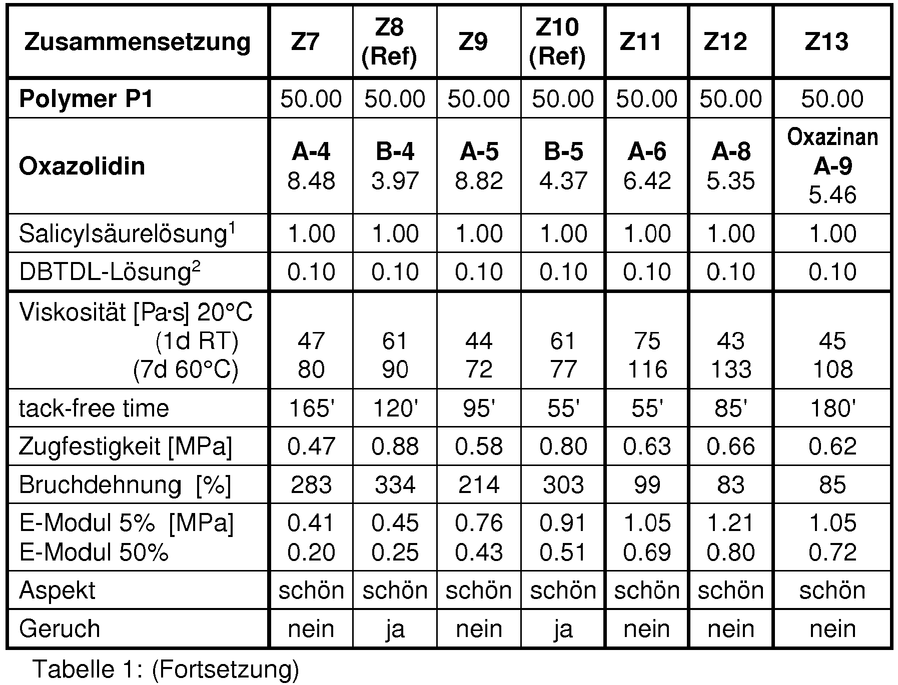

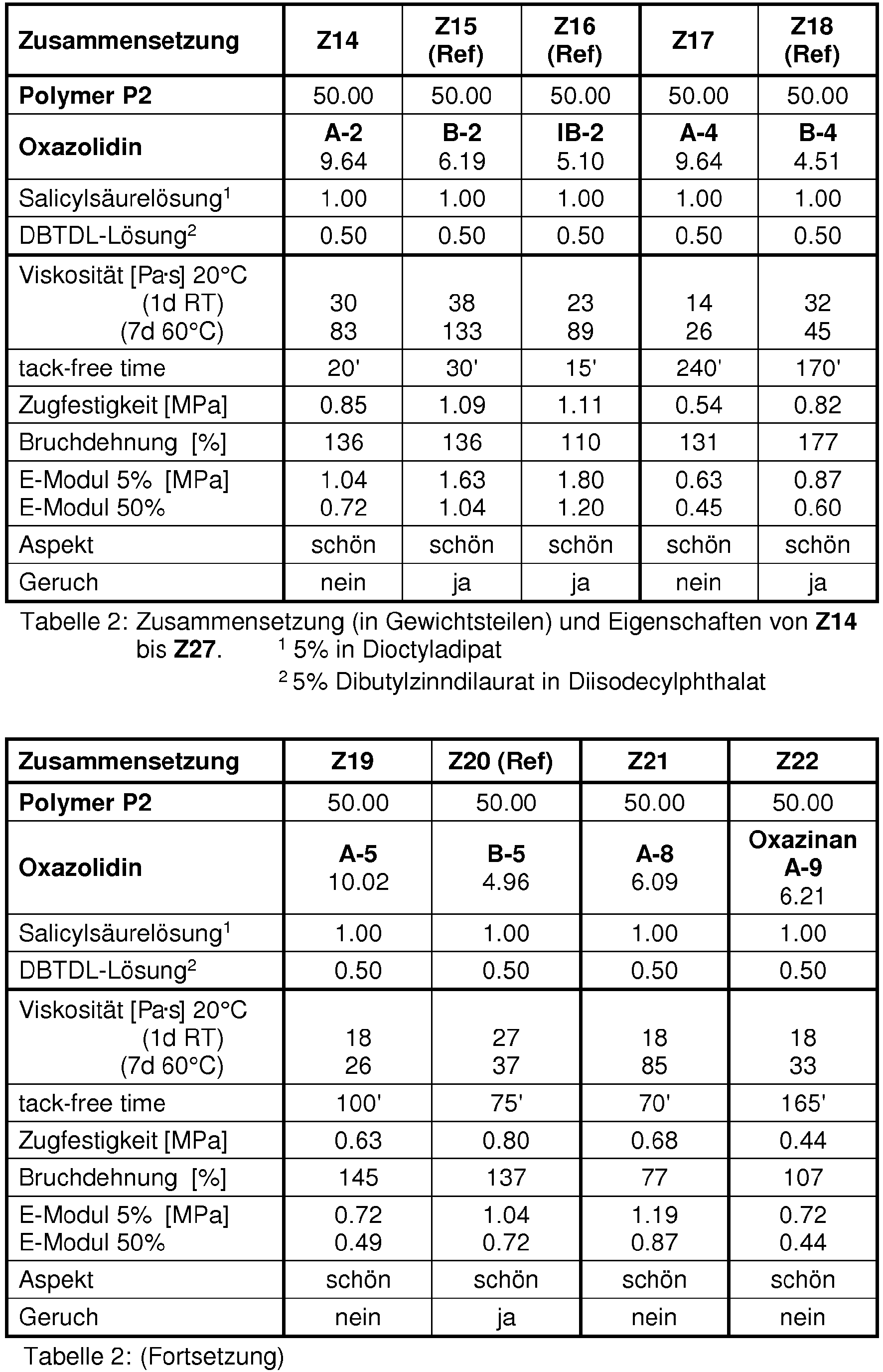

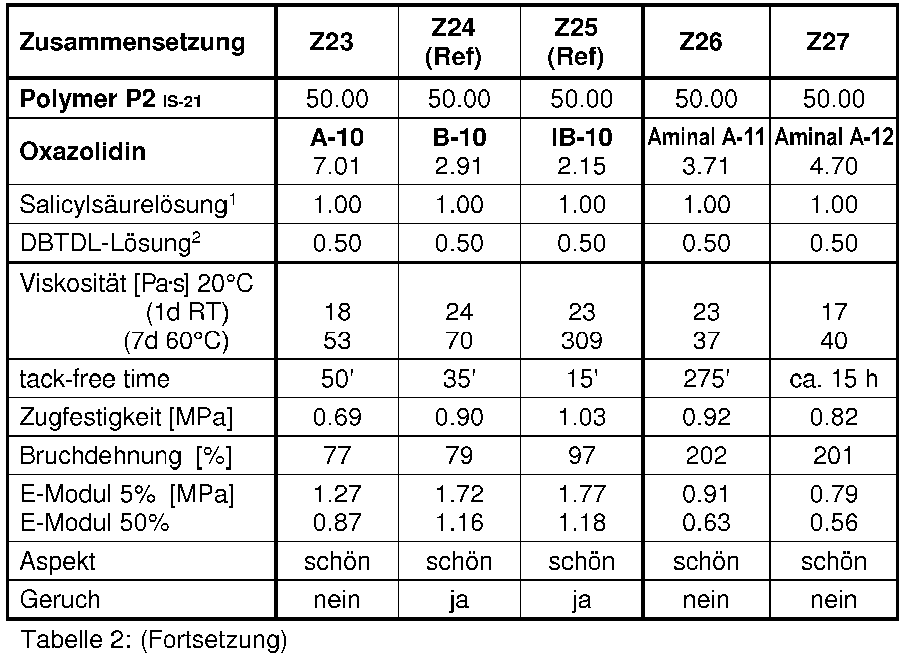

### Zusammensetzungen Z28 bis Z32:

Für jede Zusammensetzung wurden die in der Tabelle 3 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt.

Die verwendete **Vormischung-1** wurde hergestellt, indem 24.0 Gewichtsteile **Polymer P3,** 2.5 Gewichtsteile Diisodecylphthalat, 40.8 Gewichtsteile Kreide, 28 Gewichtsteile Verdickungsmittel, 4.5 Gewichtsteile Titandioxid und 0.2 Gewichtsteile p-Tosylisocyanat unter Feuchtigkeitsausschluss mittels des Zentrifugalmischers vermischt und unter Feuchtigkeitsausschluss aufbewahrt wurden.

Das Verdickungsmittel wurde vorgängig hergestellt, indem in einem Vakuummischer 300 g Diisodecylphthalat und 48 g 4,4'-Diphenylmethandiisocyanat (Desmodur^{®} 44 MC L, von Covestro) vorgelegt, leicht aufgewärmt und anschliessend unter starkem Rühren 27 g Monobutylamin langsam zugetropft wurde. Die entstehende Paste wurde unter Vakuum und Kühlung eine Stunde weitergerührt.

Jede Zusammensetzung wurde geprüft wie für Zusammensetzung **Z1** beschrieben.

Die Resultate sind in der Tabelle 3 angegeben.

Bei den mit (Ref) gekennzeichneten Zusammensetzungen handelt es sich um Vergleichsbeispiele.

**Tabelle 3: Zusammensetzung (in Gewichtsteilen) und Eigenschaften von Z28 bis Z32. ¹ 5% in Dioctyladipat ² 5% Dibutylzinndilaurat in Diisodecylphthalat**

| **Zusammensetzung** | **Z28** | **Z29** | **Z30** | **Z31** | **Z32 (Ref)** |
|---|---|---|---|---|---|
| **Vormischung-1** | 80.00 | 80.00 | 80.00 | 80.00 | 80.00 |
| **Oxazolidin** | **A-4** | **A-5** | **A-6** | **A-7** | |
| | 2.69 | 2.81 | 1.34 | 4.30 | - |
| **Aldimin C-1** | - | - | 1.47 | 1.47 | 2.05 |
| Salicylsäurelösung¹ | 0.40 | 0.40 | 0.40 | 0.40 | 0.40 |
| DBTDL-Lösung² | 0.10 | 0.10 | 0.10 | 0.10 | 0.10 |
| Viskosität [Pa·s] 20°C (1d RT) | 118 | 110 | 160 | 101 | 168 |
| tack-free time | 100' | 75' | 35' | 40' | 25' |
| Zugfestigkeit [MPa] | 1.43 | 1.51 | 1.40 | 1.04 | 1.70 |
| Bruchdehnung [%] | 797 | 845 | 858 | 938 | 823 |
| E-Modul 5% [MPa] | 1.13 | 1.13 | 1.02 | 0.75 | 1.43 |
| E-Modul 50% | 0.60 | 0.65 | 0.54 | 0.41 | 0.67 |
| Aspekt | schön | schön | schön | schön | schön |
| Geruch | nein | nein | nein | nein | nein |

### Zusammensetzungen Z33 bis Z36:

Für jede Zusammensetzung wurden die in der Tabelle 4 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt.

Jede Zusammensetzung wurde folgendermassen geprüft:
Als Mass für die Weichmachermigration wurde die Fleckenbildung auf Karton bestimmt. Dazu wurde jede Zusammensetzung so auf ein Stück Pappkarton appliziert, dass sie eine runde Grundfläche von 15 mm Durchmesser und eine Höhe von 4 mm aufwies, und während 7 Tagen im NK gelagert. Um jede Zusammensetzung herum war danach auf dem Karton ein dunkler ovaler Fleck entstanden. Dessen Ausmasse (Höhe und Breite) wurden ausgemessen und in der Tabelle 4 als **Migration** angegeben.

Bei den mit (Ref) gekennzeichneten Zusammensetzungen handelt es sich um Vergleichsbeispiele.

**Tabelle 4: Zusammensetzung (in Gewichtsteilen) und Eigenschaften von Z33 bis Z36.. ¹ 5% in Dioctyladipat ² 5% Dibutylzinndilaurat in Diisodecylphthalat**

| **Zusammensetzung** | **Z33** | **Z34 (Ref)** | **Z35 (Ref)** | **Z36 (Ref)** |
|---|---|---|---|---|
| **Polymer P4** | 15.00 | 15.00 | 15.00 | 15.00 |
| Kreide | 15.00 | 15.00 | 15.00 | 15.00 |
| Kieselsäure | 1.13 | 1.13 | 1.13 | 1.13 |
| | **Oxazolidin** | **Oxazolidin** | **Aldimin** | |
| **Härter** | **A-2** | **B-2** | **C-1** | - |
| | 7.65 | 4.61 | 5.46 | |
| Salicylsäurelösung¹ | 3.00 | 3.00 | 3.00 | 3.00 |
| DBTDL-Lösung² | 1.50 | 1.50 | 1.50 | 1.50 |
| **Migration** Höhe **(7d)** [mm] Breite | 20 | 19 | 33 | 19 |
| | 21 | 19 | 28 | 19 |
| **Geruch** | nein | ja | nein | nein |

### Verwendung von Oxazolidin A-13 als Trocknungsmittel und/oder Härter:

### Zusammensetzungen Z37 bis Z40:

Für jede Zusammensetzung wurden 105.0 Gewichtsteile **Polymer P5,** 60.0 Gewichtsteile des für Zusammensetzung **Z28** beschriebenen Verdickungsmittels, 30.0 Gewichtsteile Diisodecylphthalat, 100.0 Gewichtsteile Kreide, 7.0 Gewichtsteile Kieselsäure (Aerosil^{®} R972, von Evonik), die in der Tabelle 5 angegebenen Gewichtsteile Oxazolidin **A-13** oder Incozol 2 (von Incorez), sowie 2.0 Gewichtsteile Salicylsäurelösung (5 Gewichts-% in Dioctyladipat) und 1.0 Gewichtsteile DBTDL-Lösung (5 Gewichts-% Dibutylzinndilaurat in Diisodecylphthalat) unter Feuchtigkeitsausschluss mittels des Zentrifugalmischers vermischt und unter Feuchtigkeitsausschluss aufbewahrt.

Die Zusammensetzungen wurden folgendermassen geprüft:
Als Mass für die Lagerstabilität wurden die **Viskosität (1d RT)** am Folgetag der Herstellung, die **Viskosität (7d RT)** nach einer Lagerung im verschlossenen Gebinde während 7 Tagen bei Raumtemperatur und die **Viskosität (7d 60°C)** nach einer Lagerung im verschlossenen Gebinde während 7 Tagen in einem 60 °C warmen Umluftofen bestimmt, wie vorgängig beschrieben.

Als Mass für die Offenzeit wurde die Hautbildungszeit **(tack-free time)** bestimmt, wie vorgängig beschrieben.

Die mechanischen Eigenschaften **Zugfestigkeit** (Bruchkraft), **Bruchdehnung,** und **E-Modul 5%** (bei 0.5-5% Dehnung) und **E-Modul 25%** (bei 0.5-25% Dehnung) wurden bestimmt wie vorgängig beschrieben.

Die **Shore A** Härte wurde bestimmt nach DIN 53505 an während 7 Tagen im Nomklima gehärteten Prüfkörpern.

Die Resultate sind in der Tabelle 5 angegeben.

Bei den mit (Ref) gekennzeichneten Zusammensetzungen handelt es sich um Vergleichsbeispiele.

**Tabelle 5: Zusammensetzung und Eigenschaften von Z37 bis Z40. "GT" steht für Gewichtsteile**

| **Zusammensetzung** | | **Z37** | **Z38 (Ref)** | **Z39** | **Z40 (Ref)** |
|---|---|---|---|---|---|
| | | mit 2.7 GT Oxazolidin **A13** | mit 1.6 GT Incozol 2 | mit 7.5 GT Oxazolidin **A13** | mit 4.4 GT Incozol 2 |
| Viskosität [Pa·s] | | | | | |
| 20°C | (1d RT) | 152 | 168 | 141 | 159 |
| | (7d RT) | 161 | 171 | 144 | 218 |
| | (7d 60°C) | 188 | 264 | 189 | 516 |
| tack-free time | | 120' | 75' | 150' | 80' |
| Zugfestigkeit [MPa] | | 1.75 | 1.84 | 1.79 | 1.95 |
| Bruchdehnung [%] | | 420 | 400 | 940 | 875 |
| E-Modul 5% [MPa] | | 3.66 | 3.65 | 1.44 | 1.63 |
| E-Modul 25% | | 2.58 | 2.61 | 1.19 | 1.39 |
| Shore A | | 53 | 56 | 31 | 35 |

### Verwendung von Oxazolidin A-14 als Haftvermittler:

### Aktivator-1 :

2.5 g Oxazolidin **A-14** wurden in 250 g trockenem Ethylacetat gelöst und unter Ausschluss von Feuchtigkeit aufbewahrt.

### Zusammensetzung Z-41:

1.5 g Oxazolidin **A-14** wurden unter Stickstoffatmosphäre mit 150 g Polymer **P1** vermischt und unter Ausschluss von Feuchtigkeit aufbewahrt.

Als Mass für die Wirkung als Haftvermittler wurde eine Glasplatte (Floatglas; Firma Rocholl, Schönbrunn, Deutschland) mit den Massen 10 × 15 cm auf der Luftseite längsseitig so mit Distanzband-beklebt, dass drei Glasbahnen von je 2 x 8 cm erhalten wurden. Die erste und die zweite Bahn wurden jeweils einmal mit einem mit Ethylacetat benetzten Hygienetuch abgewischt. Die dritte Bahn wurde einmal mit einem mit dem **Aktivator-1** benetzten Hygienetuch abgewischt. Anschliessend wurde die so behandelte Glasplatte während 2 h im Normklima zum Ablüften gelagert. Dann wurden auf der ersten und der dritten Bahn jeweils 6 g Polymer **P1** in einer Schichtdicke von ca. 3 mm aufgetragen. Auf der zweiten Bahn wurden 6 g der Zusammensetzung **Z-41** in einer Schichtdicke von ca. 3 mm aufgetragen.

Die so beschichtete Glasplatte wurde während 7 Tagen im Normklima aufbewahrt und anschliessend versucht, die ausgehärteten Polymerfilme von der Glasplatte zu lösen. Als "sehr gut" wurde die Haftung bezeichnet, wenn das ausgehärtete Polymer nicht vom Glasuntergrund abgezogen werden konnte. (Auch nach mehreren Schnitten quer zur Bahnrichtung bis auf den Glasuntergrund, mit welchen das Polymer vom Glas weggeschnitten wurde, und senkrecht nach oben gerichtetem Wegziehen der Polymerbahn liess sich das Polymer nicht vom Glasuntergrund lösen.) Als "keine" wurde die Haftung bezeichnet, wenn das ausgehärtete Polymer vollständig vom Glasuntergrund abgelöst werden konnte.

Die Resultate sind in der Tabelle 6 dargestellt.

**Tabelle 6**

| **Vorbehandlung** | **Polymer-Bahn** | **Haftung** |
|---|---|---|
| Ethylacetat | Polymer **P1** | keine |
| Ethylacetat | Zusammensetzung **Z-41** | sehr gut |
| **Aktivator-1** | Polymer **P1** | sehr gut |

### Primer-Zusammensetzungen Z-42:

125.0 g Sika^{®} Primer-209 N (pigmentierter Isocyanatgruppen-haltiger Primer, von Sika Schweiz AG) wurden mit 21.2 g Oxazolidin **A-14** vermischt und unter Ausschluss von Feuchtigkeit aufbewahrt.

Die Primer-Zusammensetzung **Z-42** wurde verwendet als Haftvermittler auf Glas, wobei nach einer langen Ablüftzeit von 24 Stunden bzw. 7 Tagen Sikaflex^{®}-250 DM-5 (einkomponentiger feuchtigkeitshärtender Polyurethanklebstoff, von Sika Schweiz AG) aufgetragen und nach der Aushärtung dessen Haftung geprüft wurde. Als Referenz wurde Sika^{®} Primer-209 N eingesetzt.

Für jede Prüfung wurde eine Glasplatte mit Isopropanol gereinigt, mit einem mit Sika^{®} Aktivator-100 (Haftreiniger, von Sika Schweiz AG) getränkten Hygienetuch abgewischt und nach einer Ablüftzeit von 10 min die Primer-Zusammensetzung in dünner Schicht mit einem Schwamm aufgetragen. Nach einer Ablüftzeit von 24 Stunden bzw. 7 Tagen im Normklima wurde der auf 60°C vorgewärmte Sikaflex^{®}-250 DM-5 in Form einer Dreiecksraupe von 10 mm Breite und 100 mm Länge auf die Primer-Schicht appliziert und während 7 Tagen im Normklima gelagert, wobei der applizierte Klebstoff aushärtete. Anschliessend wurde die Haftung der Klebstoff-Raupe auf der Glasplatte getestet, indem die Raupe am Ende knapp über der Klebefläche eingeschnitten, das eingeschnittene Ende der Raupe mit einer Rundzange festgehalten und versucht wurde, die Raupe vom Untergrund wegzuziehen. Dann wurde die Raupe erneut bis auf den Untergrund eingeschnitten, wobei der freigeschnittene Teil der Raupe mit der Rundzange aufgerollt und wiederum versucht wurde, die Raupe vom Untergrund abzuziehen. So wurde die Raupe auf einer Länge von 80 mm ziehend vom Untergrund weggeschnitten. Anschliessend wurde anhand des Bruchbildes die Haftung nach folgender Skala beurteilt.
1 (= sehr gut) steht für mehr als 95% Kohäsionsbruch
2 (= gut) steht für 75 bis 95% Kohäsionsbruch

Die Resultate sind in der Tabelle 7 angegeben.

**Tabelle 7**

| Primer-Zusammensetzung: | | **Z-42** | Sika^{®} Primer-209 N |
|---|---|---|---|
| Haftung von Sikaflex^{®} 250 DM-5 | Ablüftzeit: | | |
| | 24h | 1 | 2 |
| | 7d | 1 | 1 |

## Patentansprüche

1. Verbindung der Formel (I), 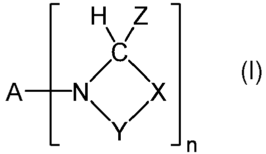 wobei
n für 1, 2 oder 3 steht,
Z für einen mit einer Alkyl- oder Alkoxy-Gruppe substituierten Aryl-Rest mit insgesamt 12 bis 26 C-Atomen steht,
A für einen n-wertigen organischen Rest mit einem Molekulargewicht im Bereich von 15 bis 10'000 g/mol steht,
X für O oder S oder NR⁰ steht, wobei R⁰ für einen einwertigen organischen Rest mit 1 bis 18 C-Atomen steht, und
Y für einen 1,2-Ethylen- oder 1,3-Propylen-Rest, welcher gegebenenfalls substituiert ist, steht,
wobei A und Y im Fall von n = 1 auch zu einem dreiwertigen Rest mit 4 bis 10 C-Atomen verbunden sein können.

2. Verbindung gemäss Anspruch 1, **dadurch gekennzeichnet, dass** A für einen n-wertigen aliphatischen, cycloaliphatischen oder arylaliphatischen, gegebenenfalls Sauerstoff- oder Stickstoff- oder Silicium-Atome aufweisenden Kohlenwasserstoff-Rest mit einem Molekulargewicht im Bereich von 15 bis 6'000 g/mol steht.

3. Verbindung gemäss einem der Ansprüche 1 oder 2, **dadurch gekennzeichnet, dass** Z für einen Rest der Formel (II) steht, 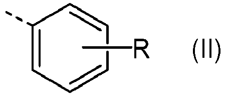 wobei R für einen Alkyl- oder Alkoxy-Rest mit 6 bis 20 C-Atomen steht.

4. Verbindung gemäss Anspruch 3, **dadurch gekennzeichnet, dass** R für einen verzweigten Rest steht.

5. Gemisch aus Verbindungen gemäss Anspruch 3, wobei R ausgewählt ist aus mehrheitlich verzweigten 4-Decyl-, 4-Undecyl-, 4-Dodecyl-, 4-Tridecyl- und 4-Tetradecyl-Resten.

6. Verbindung gemäss einem der Ansprüche 1 bis 5, **dadurch gekennzeichnet, dass** n für 1, Y für 1,2-Ethylen, X für O und A für A¹ steht, wobei A¹ für einen gegebenenfalls eine Hydroxylgruppe aufweisenden Alkyl-, Cycloalkyl- oder Aralkyl-Rest mit 1 bis 8 C-Atomen steht und diese Verbindung somit die Formel (Ia) aufweist.

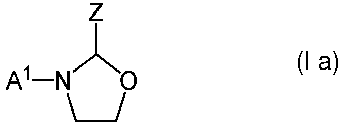

7. Verbindung gemäss einem der Ansprüche 1 bis 5, **dadurch gekennzeichnet, dass** n für 1, X für O und A für A² steht, wobei A² für einen eine Silangruppe aufweisenden Alkyl-Rest mit 1 bis 6 C-Atomen steht und diese Verbindung somit die Formel (Ib) aufweist.

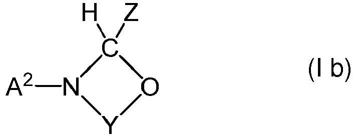

8. Verbindung gemäss einem der Ansprüche 1 bis 5, **dadurch gekennzeichnet, dass** n für 1, X für O und A für Z-CH=N-A³--- steht, wobei A³ für einen Alkylen-, Cycloalkylen- oder Arylalkylen-Rest mit 1 bis 8 C-Atomen steht und diese Verbindung somit die Formel (Ic) aufweist.

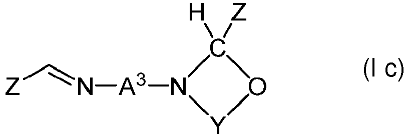

9. Verbindung gemäss einem der Ansprüche 1 bis 5, **dadurch gekennzeichnet, dass** n für 2 und A für A⁴ steht, wobei A⁴ für einen gegebenenfalls eine oder mehrere Ether-, Ester-, Carbonat- oder UrethanGruppen tragenden Alkylen- oder Cycloalkylen- oder Arylalkylen-Rest mit 2 bis 50 C-Atomen steht und diese Verbindung somit die Formel (Id) aufweist.

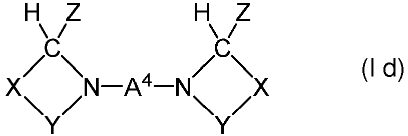

10. Verbindung gemäss einem der Ansprüche 1 bis 5, **dadurch gekennzeichnet, dass** n für 1, X für O, A für A⁵ und Y für einen Rest der Formel 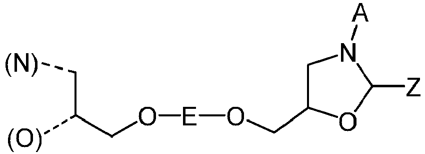 steht, wobei A⁵ für einen gegebenenfalls eine oder mehrere Ether-Gruppen tragenden Alkyl-, Cycloalkyl- oder Aralkyl-Rest mit 1 bis 35 C-Atomen und E für einen zweiwertigen, gegebenenfalls eine oder mehrere Ether-, Ester- oder Glycidoxy-Gruppen tragenden Kohlenwasserstoff-Rest mit 6 bis 20 C-Atomen stehen und diese Verbindung somit die Formel (Ie) aufweist.

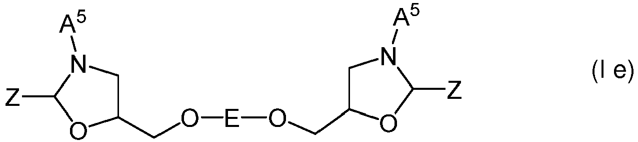

11. Verwendung von mindestens einer Verbindung gemäss einem der Ansprüche 1 bis 10 als latenter Härter für Isocyanatgruppen-haltige Zusammensetzungen.

12. Zusammensetzung umfassend
- mindestens eine Verbindung gemäss einem der Ansprüche 1 bis 10 und
- mindestens ein Polyisocyanat und/oder mindestens ein Isocyanatgruppen-haltiges Polyurethanpolymer.

13. Zusammensetzung gemäss Anspruch 14, **dadurch gekennzeichnet, dass** sie einen Klebstoff oder einen Dichtstoff oder eine Beschichtung darstellt.

14. Verwendung von mindestens einem Aldehyd der Formel (IV), 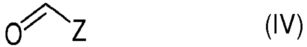 wobei Z für einen mit einer Alkyl- oder Alkoxy-Gruppe substituierten Aryl-Rest mit insgesamt 12 bis 26 C-Atomen steht,
als Blockierungsmittel für Aminoalkohole, bei welchen das Stickstoff- und das Sauerstoff-Atom durch zwei oder drei C-Atome voneinander getrennt sind.

15. Verwendung gemäss Anspruch 14, **dadurch gekennzeichnet, dass** als Aldehyd der Formel (IV) ein Gemisch enthaltend 4-Decylbenzaldehyde, 4-Undecylbenzaldehyde, 4-Dodecylbenzaldehyde, 4-Tridecylbenzaldehyde und 4-Tetradecylbenzaldehyde, deren Alkyl-Reste mehrheitlich verzweigt sind, verwendet wird.

## Claims

1. Compound of the formula (I) 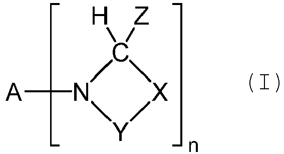 where
n is 1, 2 or 3,
Z is an aryl radical substituted by an alkyl or alkoxy group and having a total of 12 to 26 carbon atoms,
A is an n-valent organic radical having a molecular weight in the range from 15 to 10 000 g/mol,
X is O or S or NR⁰ where R⁰ is a monovalent organic radical having 1 to 18 carbon atoms and
Y is a 1,2-ethylene or 1,3-propylene radical which is optionally substituted,
where A and Y in the case that n = 1 may also be bonded to a trivalent radical having 4 to 10 carbon atoms.

2. Compound according to Claim 1, **characterized in that** A is an n-valent aliphatic, cycloaliphatic or arylaliphatic hydrocarbyl radical optionally having oxygen or nitrogen or silicon atoms and having a molecular weight in the range from 15 to 6000 g/mol.

3. Compound according to either of Claims 1 and 2, **characterized in that** Z is a radical of the formula (II) 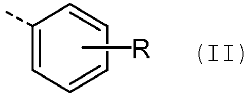 where R is an alkyl or alkoxy radical having 6 to 20 carbon atoms.

4. Compound according to Claim 3, **characterized in that** R is a branched radical.

5. Mixture of compounds according to Claim 3, wherein R is selected from mainly branched 4-decyl, 4-undecyl, 4-dodecyl, 4-tridecyl and 4-tetradecyl radicals.

6. Compound according to any of Claims 1 to 5, **characterized in that** n is 1, Y is 1,2-ethylene, X is O and A is A¹ where A¹ is an alkyl, cycloalkyl or aralkyl radical optionally having a hydroxyl group and having 1 to 8 carbon atoms and this compound thus has the formula (Ia).

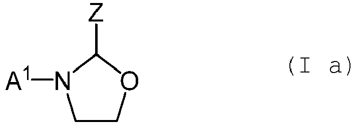

7. Compound according to any of Claims 1 to 5, **characterized in that** n is 1, X is O and A is A² where A² is an alkyl radical having a silane group and having 1 to 6 carbon atoms and this compound thus has the formula (I b).

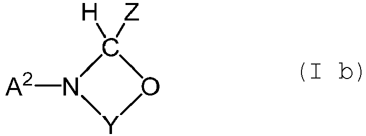

8. Compound according to any of Claims 1 to 5, **characterized in that** n is 1, X is O and A is **Z-CH=N-A³---** where A³ is an alkylene, cycloalkylene or arylalkylene radical having 1 to 8 carbon atoms and this compound thus has the formula (I c).

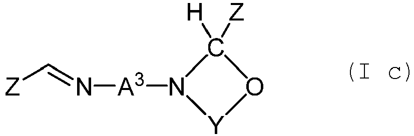

9. Compound according to any of Claims 1 to 5, **characterized in that** n is 2 and A is A⁴ where A⁴ is an alkylene or cycloalkylene or arylalkylene radical optionally bearing one or more ether, ester, carbonate or urethane groups and having 2 to 50 carbon atoms and this compound thus has the formula (I d).

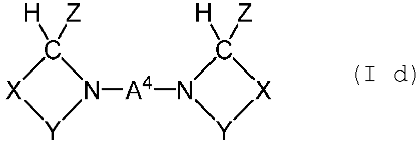

10. Compound according to any of Claims 1 to 5, **characterized in that** n is 1, X is O, A is A⁵ and Y is a radical of the formula 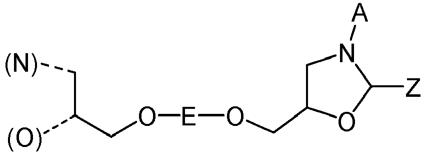 where A⁵ is an alkyl, cycloalkyl or aralkyl radical optionally bearing one or more ether groups and having 1 to 35 carbon atoms and E is a divalent hydrocarbyl radical optionally bearing one or more ether, ester or glycidoxy groups and having 6 to 20 carbon atoms and this compound thus has the formula (I e).

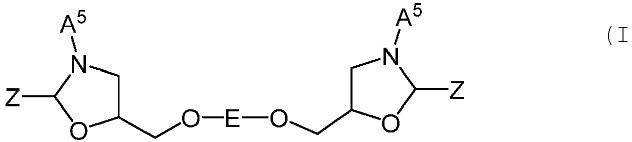

11. The use of at least one compound according to any of Claims 1 to 10 as latent curing agent for compositions containing isocyanate groups.

12. Composition comprising
- at least one compound according to any of Claims 1 to 10 and
- at least one polyisocyanate and/or at least one polyurethane polymer containing isocyanate groups.

13. Composition according to Claim 14, **characterized in that** it is an adhesive or a sealant or a coating.

14. Use of at least one aldehyde of the formula (IV) 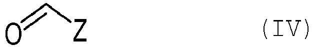 where Z is an aryl radical substituted by an alkyl or alkoxy group and having a total of 12 to 26 carbon atoms,
as blocking agent for amino alcohols, in which the nitrogen atom and the oxygen atom are separated from one another by two or three carbon atoms.

15. Use according to Claim 14, **characterized in that** the aldehyde of the formula (IV) used is a mixture comprising 4-decylbenzaldehydes, 4-undecylbenzaldehydes, 4-dodecylbenzaldehydes, 4-tridecylbenzaldehydes and 4-tetradecylbenzaldehydes, the alkyl radicals of which are mainly branched.

## Revendications

1. Composé de formule (I), 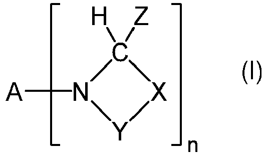 dans laquelle
n vaut 1, 2 ou 3,
Z représente un radical aryle substitué par un groupe alkyle ou alcoxy, comprenant au total 12 à 26 atomes de carbone,
A représente un radical organique n-valent présentant un poids moléculaire dans la plage de 15 à 10.000 g/mole,
X représente O ou S ou NR⁰, R⁰ représentant un radical organique monovalent, comprenant 1 à 18 atomes de carbone et
Y représente un radical 1,2-éthylène ou 1,3-propylène, qui est le cas échéant substitué,
A et Y dans le cas de n = 1 pouvant également être liés en un radical trivalent comprenant 4 à 10 atomes de carbone.

2. Composé selon la revendication 1, **caractérisé en ce que** A représente un radical hydrocarboné n-valent, aliphatique, cycloaliphatique ou arylaliphatique, présentant le cas échéant des atomes d'oxygène, d'azote ou de silicium, présentant un poids moléculaire dans la plage de 15 à 6000 g/mole.

3. Composé selon l'une quelconque des revendications 1 ou 2, **caractérisé en ce que** Z représente un radical de formule (II) 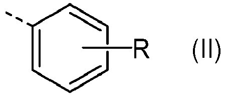 dans laquelle R représente un radical alkyle ou alcoxy comprenant 6 à 20 atomes de carbone.

4. Composé selon la revendication 3, caractérisé en ce qui R représente un radical ramifié.

5. Mélange de composés selon la revendication 3, R étant choisi parmi les radicaux 4-décyle, 4-undécyle, 4-dodécyle, 4-tridécyle et 4-tétradécyle à ramifications multiples.

6. Composé selon l'une quelconque des revendications 1 à 5, **caractérisé en ce que** n vaut 1, Y représente 1,2-éthylène, X représente O et A représente A¹, A¹ représentant un radical alkyle, cycloalkyle ou aralkyle comprenant 1 à 8 atomes de carbone, présentant le cas échéant un groupe hydroxyle et ce composé présentant donc la formule (I a).

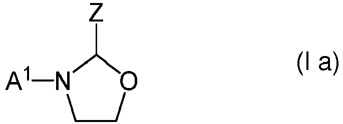

7. Composé selon l'une quelconque des revendications 1 à 5, **caractérisé en ce que** n vaut 1, X représente O et A représente A², A² représentant un radical alkyle comprenant 1 à 6 atomes de carbone, présentant un groupe silane et ce composé présentant donc la formule (I b).

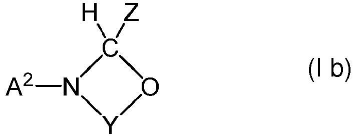

8. Composé selon l'une quelconque des revendications 1 à 5, **caractérisé en ce que** n vaut 1, X représente O et A représente Z-CH=N-A³---, A³ représentant un radical alkylène, cycloalkylène ou arylalkylène comprenant 1 à 8 atomes de carbone et ce composé présentant donc la formule (I c).

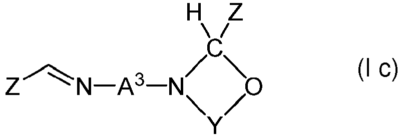

9. Composé selon l'une quelconque des revendications 1 à 5, **caractérisé en ce que** n vaut 2 et A représente A⁴, A⁴ représentant un radical alkylène, cycloalkylène ou arylalkylène comprenant 2 à 50 atomes de carbone, portant le cas échéant un ou plusieurs groupes éther, ester, carbonate ou uréthane et ce composé présentant donc la formule (I d).

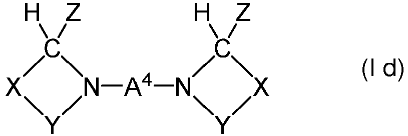

10. Composé selon l'une quelconque des revendications 1 à 5, **caractérisé en ce que** n vaut 1, X représente O, A représente A⁵ et Y représente un radical de formule 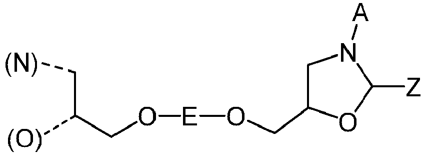 A⁵ représentant un radical alkyle, cycloalkyle ou aralkyle comprenant 1 à 35 atomes de carbone, portant le cas échéant un ou plusieurs groupes éther et E représentant un radical hydrocarboné divalent, comprenant 6 à 20 atomes de carbone, portant le cas échéant un ou plusieurs groupes éther, ester ou glycidoxy et ce composé présentant donc la formule (I e).

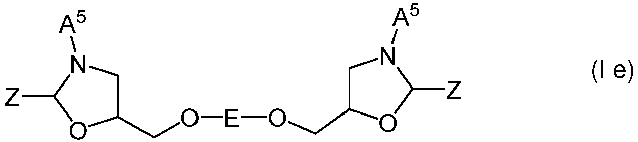

11. Utilisation d'au moins un composé selon l'une quelconque des revendications 1 à 10 en tant que durcisseur latent pour des compositions contenant des groupes isocyanate.

12. Composition comprenant
- au moins un composé selon l'une quelconque des revendications 1 à 10 et
- au moins un polyisocyanate et/ou au moins un polymère de polyuréthane contenant des groupes isocyanate.

13. Composition selon la revendication 14, **caractérisée**
**en ce qu'**elle représente un adhésif ou un matériau d'étanchéité ou un revêtement.

14. Utilisation d'au moins un aldéhyde de formule (IV) 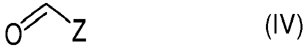 Z représentant un radical aryle substitué par alkyle ou alcoxy comprenant au total 12 à 26 atomes de carbone, comme agent de blocage pour des aminoalcools dans lesquels l'atome d'azote et l'atome d'oxygène sont séparés l'un de l'autre par deux ou trois atomes de carbone.

15. Utilisation selon la revendication 14, **caractérisée**
**en ce qu'**on utilise, comme aldéhyde de formule (IV), un mélange contenant du 4-décylbenzaldéhyde, du 4-undécylbenzaldéhyde, du 4-dodécylbenzaldéhyde, du 4-tridécylbenzaldéhyde et du 4-tétradécylbenzaldéhyde, dont les radicaux alkyle sont à ramifications multiples.
